(19) Europäisches Patentamt
European Patent Office
Office européen des brevets

(11) EP 0 773 918 B1

(12) **FASCICULE DE BREVET EUROPEEN**

(45) Date de publication et mention de la délivrance du brevet:
**17.07.2002 Bulletin 2002/29**

(21) Numéro de dépôt: **96917542.1**

(22) Date de dépôt: **24.05.1996**

(51) Int Cl.7: **C07C 41/30**, C07C 45/38, C07C 43/23, C07C 47/575

(86) Numéro de dépôt international:
**PCT/FR96/00778**

(87) Numéro de publication internationale:
**WO 96/37452 (28.11.1996 Gazette 1996/52)**

# (54) PROCEDE D'HYDROXYALKYLATION D'UN ETHER AROMATIQUE CARBOCYCLIQUE

VERFAHREN ZUR HYDROXYALKYLIERUNG VON CARBOCYCLISCHEN AROMATISCHEN ETHERN

METHOD FOR HYDROXYALKYLATING A CARBOCYCLIC AROMATIC ETHER

(84) Etats contractants désignés:
**BE CH DE ES FR GB IT LI NL**

(30) Priorité: **24.05.1995 FR 9506187**

(43) Date de publication de la demande:
**21.05.1997 Bulletin 1997/21**

(73) Titulaire: **RHODIA CHIMIE**
**92408 Courbevoie Cédex (FR)**

(72) Inventeurs:
- **MOREAU, Claude**
  **F-34880 Laverune (FR)**
- **RAZIGADE-TROUSSELIER, Sylvie**
  **F-34179 Castelnau-le-Lez (FR)**
- **FINIELS, Annie**
  **F-34090 Montpellier (FR)**
- **FAZULA, François**
  **F-34820 Teyran (FR)**
- **GILBERT, Laurent**
  **F-75015 Paris (FR)**

(74) Mandataire: **Dutruc-Rosset, Marie-Claude et al**
**Rhodia Services,**
**Direction de la Propriété Industrielle,**
**40, rue de la Haie-Coq**
**93306 Aubervilliers Cedex (FR)**

(56) Documents cités:
**EP-A- 0 485 613**
**EP-A- 0 667 331**
**FR-A- 2 305 420**
**FR-A- 2 350 323**
**US-A- 3 496 239**
**US-A- 3 673 257**

- **DATABASE WPI Week 8905 Derwent Publications Ltd., London, GB; AN 89-035249 XP002013492 & JP,A,63 307 835 (MITSUI TOATSU)**
- **STUDIES IN SURFACE SCIENCE AND CATALYSIS, vol. 78, 1993, pages 567-574, XP002013490 M. H. BURGERS ET AL: "Aromatic hydroxyalkylation using (silico)aluminophosphate molecular sieves"**
- **CHEMICAL ABSTRACTS, vol. 93, no. 19, 1980 Columbus, Ohio, US; abstract no. 185964d, page 624; colonne 1; XP002013491 & JP,A,08 059 128 (UBE INDUSTRIES)**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 773 918 B1

## Description

La présente invention a pour objet un procédé de préparation d'un éther aromatique carbocyclique hydroxyalkylé et dérivé.

L'invention vise préférentiellement la préparation de l'alcool 3-méthoxy-4-hydroxybenzylique appelé "p-vanillole", par hydroxyméthylation du gaïacol.

Elle concerne également l'oxydation des éthers hydroxyalkylés obtenus et notamment l'oxydation de l'alcool 3-méthoxy-4-hydroxybenzylique en 3-méthoxy-4-hydroxybenzaldéhyde dénommé couramment "vanilline".

Il est connu selon EP-A 0 485 613 d'effectuer une para-hydroxyméthylation d'un phénol, notamment du gaïacol par réaction de ce dernier avec du formaldéhyde, dans un solvant organique alcoolique, et en présence d'un ammonium quaternaire tel que par exemple, l'hydroxyde de tétraméthylammonium.

En plus de la nécessité de travailler en milieu alcoolique anhydre, l'inconvénient majeur de ce procédé est de faire appel à un ammonium quaternaire qu'il est nécessaire de récupérer en fin de réaction, en raison de son coût élevé.

La présente invention propose un nouveau procédé faisant appel à une catalyse hétérogène permettant d'obvier aux inconvénients précités.

Il a maintenant été trouvé et c'est ce qui constitue l'objet de la présente invention, un procédé de préparation d'un éther aromatique carbocyclique hydroxyalkylé et dérivé qui consiste à faire réagir ledit éther aromatique avec un composé carbonylé, en présence d'un catalyseur, ledit procédé étant caractérisé par le fait que l'on conduit la réaction d'hydroxyalkylation, à une température inférieure ou égale à 100°C, en présence d'une quantité efficace d'une zéolithe.

L'invention concerne les éthers aromatiques carbocycliques.

Dans l'exposé qui suit de la présente invention, on entend "par éther aromatique carbocyclique", un carbocycle aromatique dont un atome d'hydrogène directement lié au noyau aromatique est remplacé par un groupe éther et par "aromatique", la notion classique d'aromaticité telle que définie dans la littérature, notamment par Jerry MARCH, Advanced Organic Chemistry, 4ème édition, John Wiley and Sons, 1992, pp. 40 et suivantes.

Plus précisément, la présente invention a pour objet un procédé d'hydroxyalkylation d'un éther aromatique carbocyclique de formule générale (I) :

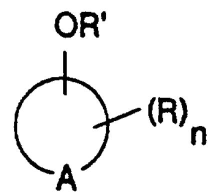

(I)

dans laquelle :

- A symbolise le reste d'un cycle formant tout ou partie d'un système carbocyclique aromatique, monocyclique ou polycyclique, système comprenant au moins un groupe OR' : ledit reste cyclique pouvant porter un ou plusieurs substituants,
- R représente un ou plusieurs substituants, identiques ou différents,
- R' représente un radical hydrocarboné ayant de 1 à 24 atomes de carbone, qui peut être un radical aliphatique acyclique saturé ou insaturé, linéaire ou ramifié ; un radical cycloaliphatique saturé, insaturé ou aromatique, monocyclique ou polycyclique ; un radical aliphatique saturé ou insaturé, linéaire ou ramifié, porteur d'un substituant cyclique,
- R' et R peuvent former un cycle comprenant éventuellement un autre hétéroatome,
- n est un nombre inférieur ou égal à 4.

Dans le présent texte, on désigne, de manière simplifiée, par "groupes alkoxy", les groupes du type -O-R' dans lesquels R' a la signification donnée précédemment. R' représente donc aussi bien un radical aliphatique acyclique ou cycloaliphatique, saturé, insaturé ou aromatique qu'un radical aliphatique saturé ou insaturé porteur d'un substituant cyclique.

L'éther aromatique carbocyclique qui intervient dans le procédé de l'invention répond à la formule (I) dans laquelle R' représente un radical aliphatique acyclique, saturé ou insaturé, linéaire ou ramifié.

Plus préférentiellement, R' représente un radical alkyle linéaire ou ramifié ayant de 1 à 12 atomes de carbone, de préférence de 1 à 6 atomes de carbone : la chaîne hydrocarbonée pouvant être éventuellement interrompue par

un hétéroatome (par exemple, l'oxygène), par un groupe fonctionnel (par exemple -CO-) et/ou porteuse d'un substituant (par exemple, un halogène).

Le radical aliphatique acyclique, saturé ou insaturé, linéaire ou ramifié peut être éventuellement porteur d'un substituant cyclique. Par cycle, on entend de préférence, un cycle carbocyclique saturé, insaturé ou aromatique, de préférence cycloaliphatique ou aromatique notamment cycloaliphatique comprenant 6 atomes de carbone dans le cycle ou benzénique.

Le radical aliphatique acyclique peut être relié au cycle par un lien valentiel, un hétéroatome ou un groupe fonctionnel et des exemples sont donnés ci-dessus.

Le cycle peut être éventuellement substitué et à titre d'exemples de substituants cycliques, on peut envisager, entre autres, les substituants tels que R dont la signification est précisée pour la formule (Ia).

R' peut représenter également un radical carbocyclique saturé ou comprenant 1 ou 2 insaturations dans le cycle, ayant généralement de 3 à 8 atomes de carbone, de préférence, 6 atomes de carbone dans le cycle ; ledit cycle pouvant être substitué avec des substituants tels que R.

R' peut représenter également un radical carbocyclique aromatique, de préférence monocyclique ayant généralement au moins 4 atomes de carbone, de préférence, 6 atomes de carbone dans le cycle ; ledit cycle pouvant être substitué avec des substituants tels que R.

Le procédé de l'invention s'applique tout particulièrement aux éthers aromatiques de formule (I) dans laquelle R' représente un radical alkyle linéaire ou ramifié ayant de 1 à 4 atomes de carbone ou un radical phényle.

Comme exemples de radicaux R' préférés selon l'invention, on peut citer les radicaux méthyle ou éthyle.

Dans la formule générale (I) des éthers aromatiques, le reste A peut représenter le reste d'un composé carbocyclique aromatique, monocyclique ayant au moins 4 atomes de carbone et de préférence 6 atomes de carbone ou le reste d'un composé carbocyclique polycyclique qui peut être constitué par au moins 2 carbocycles aromatiques et formant entre eux des systèmes ortho- ou ortho- et péricondensés ou par au moins 2 carbocycles dont au moins l'un d'entre eux est aromatique et formant entre eux des systèmes ortho- ou ortho- et péricondensés. On peut citer plus particulièrement, un reste naphtalénique.

Le reste A peut porter un ou plusieurs substituants sur le noyau aromatique.

Des exemples de substituants R sont donnés ci-après dans la formule (Ia) mais cette liste ne présente pas de caractère limitatif. N'importe quel substituant peut être présent sur le cycle dans la mesure où il n'interfère pas au niveau du produit désiré.

Le procédé de l'invention s'applique plus particulièrement, aux éthers aromatiques de formule (Ia) :

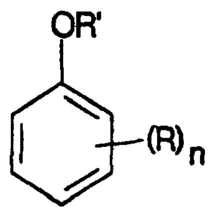

(Ia)

dans laquelle :

- n est un nombre inférieur ou égal à 4, de préférence égal à 0, 1 ou 2,
- le radical R' représente un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle ou phényle,
- le ou les radicaux R représentent l'un des atomes ou groupes suivants :
  - . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  - . un radical alcényle linéaire ou ramifié ayant de 2 à 6 atomes de carbone, de préférence, de 2 à 4 atomes de carbone, tel que vinyle, allyle,
  - . un radical cyclohexyle ou benzyle,
  - . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  - . un groupe acyle ayant de 2 à 6 atomes de carbone,
  - . un radical de formule :

$-R_1-OH$

$-R_1-COOR_2$

$-R_1-CHO$

$-R_1-NO_2$

$-R_1-CN$

$-R_1-N-(R_2)_2$

$-R_1-CO-N-(R_2)_2$

$-R_1-X$

$-R_1-CF_3$

dans lesdites formules, $R_1$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; les radicaux $R_2$, identiques ou différents représentent un atome d'hydrogène ou un radical alkyle linéaire ou ramifié ayant de 1 à 6 atomes de carbone ; X symbolise un atome d'halogène, de préférence un atome de chlore, de brome ou de fluor.

- les radicaux R' et R et les 2 atomes successifs du cycle benzénique peuvent former entre eux, un cycle ayant de 5 à 7 atomes, comprenant éventuellement un autre hétéroatome.

Lorsque n est supérieur ou égal à 1, les radicaux R' et R et les 2 atomes successifs du cycle benzénique peuvent être liés entre eux par un radical alkylène, alcénylène ou alcénylidène ayant de 2 à 4 atomes de carbone pour former un hétérocycle saturé, insaturé ou aromatique ayant de 5 à 7 atomes de carbone. Un ou plusieurs atomes de carbone peuvent être remplacés par un autre hétéroatome, de préférence l'oxygène. Ainsi, les radicaux OR' et R peuvent représenter un radical méthylène dioxy ou éthylène dioxy.
Dans la formule (Ia), R' représente préférentiellement un radical alkyle, linéaire ou ramifié, ayant de 1 à 4 atomes de carbone, de préférence, un radical méthyle ou éthyle.
L'éther aromatique de formule (Ia) peut porter un ou plusieurs substituants R.
R représente plus préférentiellement, l'un des atomes ou groupes suivants :

. un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
. un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,
. un atome d'halogène, de préférence un atome de fluor, chlore ou brome, un radical trifluorométhyle.

Dans la formule (Ia), R représente préférentiellement un radical alkoxy, linéaire ou ramifié, ayant de 1 à 4 atomes de carbone, de préférence, un radical méthoxy ou éthoxy ou un groupe hydroxyle.
Interviennent préférentiellement dans le procédé de l'invention des éthers aromatiques de formule (I) ou (Ia) dans lesquelles :

- n est égal à 0 ou 1,
- R' représente un radical alkyle, linéaire ou ramifié, ayant de 1 à 4 atomes de carbone ou un radical phényle,

- R représente un radical alkoxy, linéaire ou ramifié, ayant de 1 à 4 atomes de carbone, de préférence, un radical méthoxy ou éthoxy, un groupe hydroxyle.
- les radicaux OR' et R forment un radical méthylène dioxy ou éthylène dioxy.

Le procédé de l'invention s'applique plus particulièrement aux éthers aromatiques de formule (Ia) dans laquelle n est égal à 1, le radical R' représente un radical alkyle ayant de 1 à 4 atomes de carbone et R représente un radical alkoxy ayant de 1 à 4 atomes de carbone ou un groupe hydroxyle.

A titre illustratif de composés répondant à la formule (I), on peut mentionner plus particulièrement :

- les monoéthers tels que l'anisole, l'éthoxybenzène (phénétole), le propoxybenzène, l'isopropoxybenzène, le butoxybenzène, l'isobutoxybenzène, le 1-méthoxynaphtalène, le 2-méthoxynaphtalène, le 2-éthoxynaphtalène ; les monoéthers substitués tels que le 2-chloroanisole, le 3-chloroanisole, le 2-bromoanisole, le 3-bromoanisole, le 2-méthylanisole, le 3-méthylanisole, le 2-éthylanisole, le 3-éthylanisole, le 2-isopropylanisole, le 3-isopropylanisole, le 2-propylanisole, le 3-propylanisole, le 2-allylanisole, le 2-butylanisole, le 3-butylanisole, le 2-benzylanisole, le 2-cyclohexylanisole, le 1-bromo-2-éthoxybenzène, le 1-bromo-3-éthoxybenzène, le 1-chloro-2-éthoxybenzène, le 1-chloro-3-éthoxybenzène, le 1-éthoxy-2-éthylbenzène, le 1-éthoxy-3-éthylbenzene, le 1-méthoxy-2-allyloxybenzène, le 2,3-diméthylanisole, le 2,5-diméthylanisole,
- les diéthers comme le vératrole, le 1,3-diméthoxybenzène, le 1,4-diméthoxybenzène, le 1,2-diéthoxy- benzène, le 1,3-diéthoxybenzène, le 1,2-dipropoxybenzène, le 1,3-dipropoxy- benzène, le 1,2-méthylènedioxybenzène, le 1,2-éthylènedioxybenzène,
- les triéthers comme le 1,2,3-triméthoxybenzène, le 1,3,5-triméthoxybenzène, le 1,3,5-triéthoxybenzène.

Les composés auxquels s'applique de manière plus particulièrement intéressante le procédé selon l'invention sont l'anisole, le phénétole, le gaïacol, le guétol, le vératrole, le 1,2-méthylènedioxybenzène, le 2-méthoxynaphtalène.

Pour ce qui est du composé carbonylé, il répond plus particulièrement à la formule générale (II) :

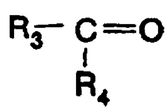

(II)

dans ladite formule (II) :

- $R_3$ et $R_4$, identiques ou différents, représentent :
  - . un atome d'hydrogène,
  - . un radical alkyle, linéaire ou ramifié, ayant de 1 à 6 atomes de carbone,
  - . un groupe alcényle, linéaire ou ramifié, ayant de 2 à 6 atomes de carbone,
  - . un groupe phényle,
  - . un groupe électro-attracteur.

Dans l'exposé qui suit de la présente invention, on entend par "groupe électro-attracteur", un groupe tel que défini par H.C. BROWN dans l'ouvrage de Jerry MARCH - Advanced Organic Chemistry, chapitre 9, pages 243 et 244.

A titre d'exemples de groupes électro-attracteurs convenant à la présente invention, on peut citer :

- un groupe -CHO,
- un groupe $-COOR_5$ dans lequel $R_5$ représente un atome d'hydrogène ou un radical alkyle linéaire ou ramifié ayant de 1 à 6 atomes de carbone,
- un groupe $-CX_2H$ dans lequel X représente un atome d'halogène, de préférence, un atome de chlore,
- un groupe $-CX_3$ dans lequel X représente un atome d'halogène, de préférence, un atome de chlore.

Comme exemples de composés carbonylés répondant à la formule (II), on peut citer :

- le formaldéhyde ou un générateur de formaldéhyde tel que, par exemple, le trioxane ou le paraformaldéhyde utilisé sous la forme de polyformaldéhydes linéaires de degré de polymérisation indifférent, ayant de préférence un nombre de motifs ($CH_2O$) compris entre 8 et 100 motifs,

- l'acide glyoxylique,
- le chloral,
- la dichloroacétone,
- l'acétaldéhyde,
- le propionaldéhyde,
- le dichloroacétaldéhyde,
- le trichloroacétaldéhyde,
- le pyruvate de méthyle,
- le pyruvate d'éthyle.

Parmi les composés carbonylés précités, le formaldéhyde est préféré.

On met en oeuvre ledit réactif généralement sous forme d'une solution aqueuse ayant une concentration inférieure à 50 % en poids, de préférence, comprise entre 20 et 50 % en poids. Il peut contenir quelques pourcentages d'un alcool, généralement le méthanol à une teneur inférieure à 5 % en poids.

Conformément au procédé de l'invention, on effectue la réaction d'hydroxyalkylation en présence d'un catalyseur constitué par une zéolithe.

Par "zéolithe", on entend un tectosilicate cristallisé d'origine naturelle ou synthétique dont les cristaux résultent de l'assemblage tridimensionnel d'unités tétraédriques de $SiO_4$ et $TO_4$ : T représentant un élément trivalent tel que aluminium, gallium, bore, fer, de préférence, l'aluminium.

Les zéolithes de type aluminosilicate sont les plus communes.

Les zéolithes présentent au sein du réseau cristallin, un système de cavités reliées entre elles par des canaux d'un diamètre bien défini que l'on appelle les pores.

Les zéolithes peuvent présenter un réseau de canaux monodimensionnel, bidimensionnel ou tridimensionnel.

Dans le procédé de l'invention, on peut faire appel à une zéolithe naturelle ou synthétique.

Comme exemples de zéolithes naturelles susceptibles d'être utilisées, on peut citer, par exemple : la chabazite, la clinoptilolite, l'érionite, la phillipsite, l'offrétite.

Conviennent tout à fait bien à la mise en oeuvre de l'invention, les zéolithes synthétiques.

Comme exemples de zéolithes synthétiques à réseau monodimensionnel, on peut citer entre autres, la zéolithe ZSM-4, la zéolithe L, la zéolithe ZSM-12, la zéolithe ZSM-22, la zéolithe ZSM-23, la zéolithe ZSM-48.

A titre d'exemples de zéolithes à réseau bidimensionnel mises en oeuvre préférentiellement, on peut mentionner la mordénite, la ferrierite.

En ce qui concerne les zéolithes à réseau tridimensionnel, on peut nommer plus particulièrement, la zéolithe β, la zéolithe Y, la zéolithe X, la zéolithe ZSM-5, la zéolithe ZSM-11, l'offrétite.

On fait appel préférentiellement aux zéolithes synthétiques et plus particulièrement aux zéolithes qui sont sous les formes suivantes:

- la mazzite de rapport molaire Si/Al de 3,4,
- la zéolithe L de rapport molaire Si/Al de 1,5 à 3,5,
- la mordénite de rapport molaire Si/Al de 5 à 150, de préférence, de 10 à 100 et encore plus préférentiellement de 10 à 25,
- la ferrierite de rapport molaire Si/Al de 3 à 10,
- l'offrétite de rapport molaire Si/Al de 4 à 8,5.
- les zéolithes β de rapport molaire Si/Al de 10 à 100, de préférence, 12 à 50,
- les zéolithes Y en particulier les zéolithes obtenues après traitement de désalumination (par exemple hydrotraitement, lavage à l'aide d'acide chlorhydrique ou traitement par $SiCl_4$) et l'on peut citer plus particulièrement les zéolithes US-Y de rapport molaire Si/Al supérieur à 3, de préférence compris entre 6 et 60 ;
- la zéolithe X de type faujasite de rapport molaire Si/Al de 0,7 à 1,5,
- les zéolithes ZSM-5 ou silicalite d'aluminium de rapport molaire Si/Al de 10 à 500,
- la zéolithe ZSM-11 de rapport molaire Si/Al de 5 à 30.

Parmi toutes ces zéolithes, on fait appel préférentiellement dans le procédé de l'invention aux zéolithes β et aux mordénites.

Les zéolithes mises en oeuvre dans le procédé de l'invention, sont des produits connus décrits dans la littérature [cf. Atlas of zeolites structure types by W. M. Meier and D. H. Olson published by the Structure Commission of the International Zeolite Association (1992)].

On peut faire appel aux zéolithes disponibles dans le commerce ou bien les synthétiser selon les procédés décrits dans la littérature.

On peut se référer à l'Atlas précité, et plus particulièrement, pour la préparation :

- de la zéolithe L à la publication de Barrer R. M. et al, Z. Kristallogr., 128, pp. 352 (1969)
- de la zéolithe ZSM-12, au brevet US 3 832 449 et à l'article LaPierre et al, Zeolites 5, pp. 346 (1985),
- de la zéolithe ZSM-22, à la publication Kokotailo G.T. et al, Zeolites 5, pp. 349 (1985),
- de la zéolithe ZSM-23, au brevet US 4 076 842 et à l'article Rohrman A. C. et al, Zeolites 5, pp. 352 (1985),
- de la zéolithe ZSM-48, aux travaux de Schlenker J. L. et al, Zeolites 5, pp. 355 (1985),
- de la zéolithe β, au brevet US 3 308 069 et à l'article Caullet P. et al, Zeolites 12, pp. 240 (1992),
- de la mordénite, aux travaux de Itabashi et al, Zeolites 6, pp. 30 (1986),
- des zéolithes X et Y respectivement aux brevets US 2 882 244 et US 3 130 007,
- de la zéolithe ZSM-5, au brevet US 3 702 886 et à l'article Shiralkar V. P. et al, Zeolites 9, pp. 363 (1989),
- de la zéolithe ZSM-11, aux travaux de Harrison I. D. et al, Zeolites 7, pp. 21(1987).

Afin de mettre en oeuvre une zéolithe présentant le rapport atomique Si/Al souhaité, il peut être nécessaire d'effectuer un traitement de désalumination.

Ainsi, on peut mettre en oeuvre les méthodes connues de l'homme du métier parmi lesquelles on peut citer, à titre d'exemples, et de manière non exhaustive, les calcinations en présence de vapeur, les calcinations en présence de vapeur d'eau suivies d'attaques par des acides minéraux ($HNO_3$, HCl...), les traitements directes de désalumination par des réactifs tels que le tétrachlorure de silicium ($SiCl_4$), l'hexafluorosilicate d'ammonium ($(NH_4)_2SiF_6$), l'éthylènediaminetétracétique (EDTA) ainsi que sa forme mono- ou disodique. On peut également faire un traitement de désalumination par attaque acide directe par des solutions d'acides minéraux tels que par exemple, l'acide chlorhydrique, l'acide nitrique, l'acide sulfurique ou d'acides organiques comme notamment l'acide acétique, l'acide oxalique. Par ailleurs, toute combinaison des méthodes de désalumination précitées est aussi possible.

La zéolithe constitue la phase catalytique. Elle peut être utilisée seule ou en mélange avec une matrice minérale. Dans la description, on désignera par "catalyseur", le catalyseur réalisé entièrement en zéolithe ou en mélange avec une matrice préparée selon des techniques connues de l'Homme du métier.

A cet effet, la matrice peut être choisie parmi les oxydes de métaux, tels que les oxydes d'aluminium, de silicium et/ou de zirconium, ou encore parmi les argiles et plus particulièrement, le kaolin, le talc ou la montmorillonite.

Dans le catalyseur, la teneur en phase active représente de 5 à 100 % du poids du catalyseur.

Les catalyseurs peuvent se présenter sous différentes formes dans le procédé de l'invention : poudre, produits mis en forme tels que granulés (par exemple, extrudés ou billes), pastilles, qui sont obtenus par extrusion, moulage, compactage ou tout autre type de procédé connu. En pratique, sur le plan industriel, ce sont les formes de granulés ou de billes qui présentent le plus d'avantages tant sur le plan de l'efficacité que sur le plan de commodité de mise en oeuvre.

La zéolithe mise en oeuvre est de préférence, sous forme acide. On effectue si nécessaire un traitement qui la rend acide.

A cet effet, on fait appel aux traitements classiques.

Ainsi, on peut échanger les cations alcalins en soumettant la zéolithe à un traitement réalisé avec de l'ammoniaque conduisant ainsi à un échange du cation alcalin par un ion ammonium puis à calciner la zéolithe échangée afin de décomposer thermiquement le cation ammonium et le remplacer par un ion $H^+$.

La quantité d'ammoniaque à mettre en oeuvre est au moins égale à la quantité nécessaire pour échanger tous les cations alcalins en ions $NH_4^+$.

On met donc au moins en jeu de $10^{-5}$ à $5.10^{-3}$ mole d'ammoniaque par gramme de zéolithe.

La réaction d'échange du cation échangeable par $NH_4^+$ est effectuée à une température qui se situe entre la température ambiante et la température de reflux du milieu réactionnel. L'opération dure quelques heures et peut être répétée.

La zéolithe peut être également acidifiée en soumettant celle-ci à un traitement acide classique. Ce traitement peut être effectué par addition d'un acide tel que notamment l'acide chlorhydrique, l'acide sulfurique, l'acide nitrique, l'acide perchlorique, l'acide phosphorique et l'acide trifluorométhanesulfonique.

Selon un mode préférentiel de mise en oeuvre, la zéolithe est acidifiée par passage d'un volume d'acide présentant une normalité comprise entre 0,1 et 2 N par gramme de zéolithe comprise entre 10 ml/g et 100 ml/g. Ce passage peut être réalisé en une seule étape ou de préférence en plusieurs étapes successives.

La réaction de l'éther aromatique carbocyclique de formule (I) avec le composé carbonylé de formule (II) est conduite, de préférence, en milieu aqueux lorsque l'on fait appel au formaldéhyde. Toutefois, il est également possible de faire la réaction au sein d'un liquide organique inerte dans les conditions réactionnelles choisies.

Comme exemptes de solvants convenant à la présente invention, on peut citer en particulier les hydrocarbures aliphatiques ou aromatiques, halogénés ou non, les éther-oxydes aliphatiques, cycloaliphatiques ou aromatiques.

A titre d'exemples d'hydrocarbures aliphatiques ou cycloaliphatiques, on peut citer plus particulièrement les paraffines tels que notamment, l'hexane, l'heptane, l'octane, le nonane, le décane, l'undécane, le dodécane, le tétradécane ou le cyclohexane, et les hydrocarbures aromatiques comme notamment le benzène, le toluène, les xylènes,

le cumène, les coupes pétrolières constituées de mélange d'alkylbenzènes notamment les coupes de type Solvesso®.

En ce qui concerne les hydrocarbures halogénés aliphatiques ou aromatiques, on peut mentionner plus particulièrement, les hydrocarbures perchlorés tels que notamment le tétrachtoroéthylène, l'hexachloroéthane ; les hydrocarbures partiellement chlorés tels que le dichlorométhane, le chloroforme, le 1,2-dichloroéthane, le 1,1,1-trichloroéthane, le 1,1,2,2-tétrachloroéthane, le pentachloroéthane, le trichloroéthylène, le 1-chlorobutane, le 1,2-dichlorobutane ; le monochlorobenzène, le 1,2-dichlorobenzène, le 1,3-dichlorobenzène, le 1,4-dichlorobenzène, le 1,2,4-trichlorobenzène ou des mélanges de différents chlorobenzènes ; le bromoforme, le bromoéthane ou le 1,2-dibromoéthane ; le monobromobenzène ou des mélanges de monobromobenzène avec un ou plusieurs dibromobenzènes ; le 1-bromonaphtalène.

On peut utiliser également à titre de solvants organiques, les éther-oxydes aliphatiques, cycloaliphatiques ou aromatiques et, plus particulièrement, l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, le diméthyléther de l'éthylèneglycol (ou 1,2-diméthoxyéthane), le diméthyléther du diéthylèneglycol (ou 1,5-diméthoxy 3-oxapentane) ; l'oxyde de phényle, l'oxyde de benzyle ; le dioxane, le tétrahydrofuranne (THF).

On peut faire appel à des solvants aprotiques polaires tels que les composés nitrés comme par exemple, le nitrométhane, le nitroéthane, le 1-nitropropane, le 2-nitropropane ou leurs mélanges, le nitrobenzène ; les nitriles aliphatiques ou aromatiques comme l'acétonitrile, le propionitrile, le butanenitrile, l'isobutanenitrile, le benzonitrile, le phénylacétonitrile ; le diméthylsulfoxyde ; la tétraméthylènesulfone (sulfolane).

Les solvants préférés sont : le dichlorométhane, le tétrachlorométhane, le dioxane, l'oxyde de diéthyle, l'oxyde d'isopropyle, l'oxyde de phényle.

On peut également utiliser un mélange de solvants organiques.

La concentration de l'éther aromatique dans le milieu peut varier dans de larges limites. Ainsi elle peut être comprise entre 0,1 et 5 moles par litre de milieu et, de préférence, entre 0,2 et 2 moles par litre.

Lorsque l'on réalise le procédé en discontinu, le catalyseur peut représenter en poids par rapport à l'éther aromatique engagé, de 5 à 80 %, de préférence, de 10 à 50 %. Cependant si l'on réalise le procédé en continu, par exemple en faisant réagir un mélange de l'éther aromatique et de composé carbonylé sur un lit fixe de catalyseur, ces rapports catalyseur/éther aromatique n'ont pas de sens et à un instant donné, on peut avoir un excès pondéral de catalyseur par rapport à l'éther aromatique de départ.

La quantité de composé carbonylé de formule (II) exprimée en moles de composé carbonylé par mole d'éther aromatique de formule (I) peut, elle aussi, varier dans de larges limites. Le rapport molaire composé carbonylé de formule (II)/éther aromatique de formule (I) peut varier entre 1 et 50. La borne supérieure ne présente aucun caractère critique mais toutefois pour des raisons économiques, il n'y a aucun intérêt à la dépasser.

La zone préférée pour ledit rapport est déterminée selon la nature de la zéolithe. Ainsi, lors de la mise en oeuvre de zéolithes naturelles ou synthétiques telles que les zéolithes L, X, Y, les mordénites de rapport molaire Si/Al de 5 à 12, la ferrierite et l'offrétite, le rapport molaire composé carbonylé/éther aromatique est de préférence, de 1 à 25. Il est avantageusement choisi entre 8 et 25 pour les zéolithes synthétiques telles que la zéolithe ZSM-4, la zéolithe ZSM-5, la zéolithe ZSM-11, la zéolithe ZSM-12, la zéolithe ZSM-22, la zéolithe ZSM-23, la zéolithe ZSM-48, la zéolithe L, la zéolithe $\beta$ et les mordénites de rapport molaire Si/Al de 12 à 150.

La température de la réaction d'hydroxyalkylation est inférieure ou égale à 100°C. Pour obtenir une meilleure sélectivité de la réaction, il y a intérêt à choisir une température peu élevée. Toutefois pour conserver une activité suffisante, elle est choisie, avantageusement entre 20°C et 100°C et encore plus préférentiellement entre 40°C et 90°C.

Généralement, la réaction est conduite à pression atmosphérique mais des pressions plus élevées peuvent également convenir allant de 1 à 50 bar, de préférence, de 1 à 25 bar. On travaille sous pression autogène lorsque la température de réaction est supérieure à la température d'ébullition des réactifs et/ou des produits.

On préfère conduire la réaction sous atmosphère contrôlée de gaz inertes tels que l'azote ou les gaz rares, par exemple l'argon.

La durée de la réaction peut être très variable. Elle se situe, le plus souvent, entre 15 minutes et 10 heures, de préférence entre 30 minutes et 5 heures.

D'un point de vue pratique, le procédé peut être mis en oeuvre en discontinu ou en continu.

Selon la première variante, on peut charger le catalyseur, le composé carbonylé de formule (II), éventuellement un solvant organique puis l'on introduit l'éther aromatique. Un mode préféré de l'invention, consiste à introduire progressivement l'éther aromatique, en continu ou par fractions puis l'on porte le mélange réactionnel à la température souhaitée.

L'autre variante de l'invention consiste à conduire la réaction en continu, dans un réacteur tubulaire comportant le catalyseur solide disposé en lit fixe.

L'éther aromatique carbocyclique et le composé carbonylé sont introduits de préférence, séparément.

Ils peuvent également être introduits dans un solvant tel que mentionné précédemment.

Le temps de séjour du flux de matière sur le lit catalytique varie, par exemple, entre 15 min et 10 heures, et

de préférence, entre 30 min et 5 heures.

En fin de réaction, on récupère une phase liquide comprenant l'éther aromatique hydroxyalkylé qui peut être récupéré de manière classique, de différentes manières.

Un premier mode de séparation de l'éther aromatique hydroxyalkylé consiste à l'extraire dans un solvant organique puis à soumettre la phase organique obtenue à une oxydation. Ce mode est préféré ainsi que le suivant, lorsque le composé carbonylé mis en oeuvre est le formaldéhyde ou un précurseur de formaldéhyde.

Comme solvants convenant, on peut citer notamment, des hydrocarbures aliphatiques ou aromatiques halogénés comme par exemple le chlorométhane, le dichlorométhane, le 1,2-dichloroéthane, le chlorobenzène, les dichlorobenzènes, les trichlorobenzènes ; des esters comme les acétates de butyle, d'isopropyle, d'amyle, de cyclohexyle; des cétones telles que par exemple l'acétone, la méthyléthylcétone, la méthylisobutylcétone.

Le rapport volumique entre le solvant organique et la phase aqueuse est choisi de préférence, entre 0,5 et 1,0.

On sépare les phases aqueuse et organique.

La phase aqueuse peut être recyclée dans la réaction.

La phase organique peut être soumise directement à l'oxydation.

Une autre variante consiste mettre en contact la phase organique avec une solution aqueuse basique afin de faire une contre-extraction en phase aqueuse, du produit obtenu sous forme salifiée ; celui-ci étant ensuite oxydé. Ce mode de mise en oeuvre convient lorsque l'éther aromatique hydroxyalkylé obtenu présente également un groupe hydroxyle et l'on peut citer le cas du gaïacol.

A cet égard, on fait appel en général, comme agent alcalin, à l'hydroxyde de sodium ou de potassium. La proportion de base minérale à utiliser est comprise entre 0,5 et 3 moles d'hydroxyde de sodium ou de potassium par rapport à l'éther aromatique hydroxyalkylé à oxyder.

La concentration de ce dernier dans la solution aqueuse d'agent alcalin doit de préférence être telle que l'on évite toute précipitation et conserve une solution homogène. Elle est habituellement comprise entre 1 % et 60 % en poids, de préférence entre 2 % et 30 %.

La phase aqueuse ainsi obtenue est soumise à l'étape d'oxydation.

Une autre variante consiste à séparer l'éther aromatique hydroxyalkylé par distillation ou par recristallisation dans un solvant approprié après élimination préalable des réactifs en excès puis à remettre le produit obtenu en solution organique afin d'effectuer l'étape d'oxydation.

Un mode préféré d'oxydation de l'invention et qui constitue un autre objet de la présente invention consiste à oxyder l'éther aromatique hydroxyalkylé, en phase liquide, à l'aide d'oxygène moléculaire ou un gaz en contenant, en présence d'un catalyseur à base d'un métal M choisi parmi les métaux du groupe 8 de la classification périodique comprenant éventuellement, à titre d'activateurs des métaux tels que le cadmium, le cérium, le bismuth, le plomb, l'argent, le tellure ou l'étain.

On peut se référer, pour plus de détails sur ces catalyseurs à US-A-3 673 257, FR-A-2 305 420, FR-A-2 350 323.

Comme exemples de catalyseurs à base d'un métal du groupe 8, on peut citer le nickel, le ruthénium, le rhodium, le palladium, l'osmium, l'iridium, le platine et leurs mélanges.

On utilise de préférence, des catalyseurs de platine et/ou de palladium, pris sous toutes les formes disponibles telles que par exemple : le noir de platine, le noir de palladium, l'oxyde de platine, l'oxyde de palladium ou le métal noble lui-même déposé sur des supports divers tels que le noir de carbone, le carbonate de calcium, les alumines et silices activées ou des matériaux équivalents. Des masses catalytiques à base de noir de carbone conviennent particulièrement.

La quantité de ce catalyseur à mettre en oeuvre, exprimée en poids de métal M par rapport à celui de l'éther aromatique hydroxyalkylé peut varier de 0,01 à 4 % et, de préférence, de 0,04 à 2 %.

L'activateur peut être choisi parmi tous ceux mentionnés dans les brevets précités. De préférence, on fait appel au bismuth, au plomb et au cadmium, sous forme de métaux libres ou de cations. Dans ce dernier cas, l'anion associé n'est pas critique et on peut utiliser tous dérivés de ces métaux. De préférence, on met en oeuvre le bismuth métal ou ses dérivés.

On peut faire appel à un dérivé minéral ou organique du bismuth dans lequel l'atome de bismuth se trouve à un degré d'oxydation supérieur à zéro, par exemple égal à **2, 3**, **4** ou **5.** Le reste associé au bismuth n'est pas critique dès l'instant qu'il satisfait à cette condition. L'activateur peut être soluble ou insoluble dans le milieu réactionnel.

Des composés illustratifs d'activateurs qui peuvent être utilisés dans le procédé selon la présente invention sont : les oxydes de bismuth ; les hydroxydes de bismuth ; les sels d'hydracides minéraux tels que : chlorure, bromure, iodure, sulfure, séléniure, tellure de bismuth ; les sels d'oxyacides minéraux tels que : sulfite, sulfate, nitrite, nitrate, phosphite, phosphate, pyrophosphate, carbonate, perchlorate, antimoniate, arséniate, sélénite, séléniate de bismuth ; les sels d'oxyacides dérivés de métaux de transition tels que : vanadate, niobate, tantalate, chromate, molybdate, tungstate, permanganate de bismuth.

D'autres composés appropriés sont également des sels d'acides organiques aliphatiques ou aromatiques

tels que : acétate, propionate, benzoate, salicylate, oxalate, tartrate, lactate, citrate de bismuth ; des phénates tels que : gallate et pyrogallate de bismuth. Ces sels et phénates peuvent être aussi des sels de bismuthyle.

Comme autres composés minéraux ou organiques, on peut utiliser des combinaisons binaires du bismuth avec des éléments tels que phosphore et arsenic ; des hétéropolyacides contenant du bismuth ainsi que leurs sels ; conviennent également les bismuthines aliphatiques et aromatiques.

A titre d'exemples spécifiques, on peut citer :

- comme oxydes : BiO ; $Bi_2O_3$ : $Bi_2O_4$ ; $Bi_2O_5$.
- comme hydroxydes : $Bi(OH)_3$,
- comme sels d'hydracides minéraux : le chlorure de bismuth $BiCl_3$ ; le bromure de bismuth $BiBr_3$ ; l'iodure de bismuth $BiI_3$ ; le sulfure de bismuth $Bi_2S_3$ ; le séléniure de bismuth $Bi_2Se_3$ : le tellure de bismuth $Bi_2Te_3$,
- comme sels d'oxyacides minéraux : le sulfite basique de bismuth $Bi_2(SO_3)_3$, $Bi_2O_3$, $5H_2O$ ; le sulfate neutre de bismuth $Bi_2(SO_4)_3$ ; le sulfate de bismuthyle $(BiO)HSO_4$ ; le nitrite de bismuthyle $(BiO)NO_2$, $0{,}5H_2O$ ; le nitrate neutre de bismuth $Bi(NO_3)_3$, $5H_2O$ ; le nitrate double de bismuth et de magnésium $2Bi(NO_3)_3$, $3Mg(NO_3)_2$, $24H_2O$ ; le nitrate de bismuthyle $(BiO)NO_3$ ; le phosphite de bismuth $Bi_2(PO_3H)_3$, $3H_2O$ ; le phosphate neutre de bismuth $BiPO_4$ ; le pyrophosphate de bismuth $Bi_4(P_2O_7)_3$ ; le carbonate de bismuthyle $(BiO)_2CO_3$, $0{,}5H_2O$ ; le perchlorate neutre de bismuth $Bi(ClO_4)_3$, $5H_2O$ ; le perchlorate de bismuthyle $(BiO)ClO_4$ ; l'antimoniate de bismuth $BiSbO_4$ ; l'arséniate neutre de bismuth $Bi(AsO_4)_3$ ; l'arséniate de bismuthyle $(BiO)AsO_4$, $5H_2O$ ; le sélénite de bismuth $Bi_2(SeO_3)_3$.
- comme sels d'oxyacides dérivés de métaux de transition : le vanadate de bismuth $BiVO_4$ ; le niobate de bismuth $BiNbO_4$ : le tantalate de bismuth $BiTaO_4$ ; le chromate neutre de bismuth $Bi_2(CrO_4)$ ; le dichromate de bismuthyle $([BiO_2]_2Cr_2O_7$ ; le chromate acide de bismuthyle $H(BiO)CrO_4$ ; le chromate double de bismuthyle et de potassium $K(BiO)CrO_4$ ; le molybdate de bismuth $Bi_2(MoO_4)_3$ ; le tungstate de bismuth $Bi_2(WO_4)_3$ ; le molybdate double de bismuth et de sodium $NaBi(MoO_4)_2$ ; le permanganate basique de bismuth $Bi_2O_2(OH)MnO_4$.
- comme sels d'acides organiques aliphatiques ou aromatiques : l'acétate de bismuth $Bi(C_2H_3O_2)_3$ ; le propionate de bismuthyle $(BiO)C_3H_5O_2$ ; le benzoate basique de bismuth $C_6H_5CO_2Bi(OH)_2$ ; le salicylate de bismuthyle $C_6H_4CO_2(BiO)(OH)$ ; l'oxalate de bismuth $(C_2O_4)_3Bi_2$ ; le tartrate de bismuth $Bi_2(C_4H_4O_6)_3$, $6H_2O$ ; le lactate de bismuth $(C_6H_9O_5)OBi$, $7H_2O$ ; le citrate de bismuth $C_6H_5O_7Bi$.
- comme phénates : le gallate basique de bismuth $C_7H_7O_7Bi$ ; le pyrogallate basique de bismuth $C_6H_3(OH)_2(OBi)(OH)$.

Comme autres composés minéraux ou organiques conviennent également : le phosphure de bismuth BiP ; l'arséniure de bismuth $Bi_3As_4$ ; le bismuthate de sodium $NaBiO_3$ ; les acides bismuth-thiocyaniques $H_2[Bi(BNS)_5]$, $H_3[Bi(CNS)_6]$ et leurs sels de sodium et potassium ; la triméthylbismuthine $Bi(CH_3)_3$, la triphénylbismuthine $Bi(C_6H_5)_3$.

Les dérivés du bismuth qui sont utilisés de préférence pour conduire le procédé selon l'invention sont : les oxydes de bismuth ; les hydroxydes de bismuth ; les sels de bismuth ou de bismuthyle d'hydracides minéraux ; les sels de bismuth ou de bismuthyle d'oxyacides minéraux ; les sels de bismuth ou de bismuthyle d'acides organiques aliphatiques ou aromatiques ; et les phénates de bismuth ou de bismuthyle.

Un groupe d'activateurs qui conviennent particulièrement bien à la réalisation de l'invention est constitué par : les oxydes de bismuth $Bi_2O_3$ et $Bi_2O_4$; l'hydroxyde de bismuth $Bi(OH)_3$ ; le sulfate neutre de bismuth $Bi_2(SO_4)_3$ ; le chlorure de bismuth $BiCl_3$ ; le bromure de bismuth $BiBr_3$ ; l'iodure de bismuth $BiI_3$ ; le nitrate neutre de bismuth $Bi(NO_3)_3$, $5H_2O$ ; le nitrate de bismuthyle $BiO(NO_3)$ ; le carbonate de bismuthyle $(BiO)_2CO_3$, $O{,}5H_2O$ ; l'acétate de bismuth $Bi(C_2H_3O_2)_3$ ; le salicylate de bismuthyle $C_6H_4CO_2(BiO)(OH)$.

La quantité d'activateur utilisée, exprimée par la quantité de métal contenue dans l'activateur par rapport au poids du métal M engagé, peut varier dans de larges limites. Par exemple, cette quantité peut être aussi petite que 0,1 % et peut atteindre le poids de métal M engagé et même le dépasser sans inconvénient.

Plus particulièrement, cette quantité est choisie de manière qu'elle apporte dans le milieu d'oxydation de 10 à 900 ppm en poids de métal activateur par rapport à l'éther aromatique hydroxyalkylé. A cet égard, des quantités supérieures d'activateur de l'ordre de 900 à 1500 ppm peuvent naturellement être utilisées, mais sans avantage supplémentaire important.

La concentration pondérale de l'éther aromatique hydroxyalkylé dans la phase liquide est habituellement comprise entre 1 % et 60 %, de préférence entre 2 % et 30 %.

Pratiquement une manière d'exécuter le procédé consiste à mettre en contact avec de l'oxygène moléculaire ou un gaz en contenant, par exemple l'air, la solution renfermant l'éther aromatique hydroxyalkylé, éventuellement l'agent alcalin, le catalyseur à base de métal M, éventuellement l'activateur, selon les proportions indiquées ci-dessus.

On opère à pression atmosphérique, mais on peut aussi le cas échéant opérer sous pression entre 1 et 20 bar.

Le mélange est ensuite agité à la température désirée jusqu'à consommation d'une quantité d'oxygène correspondant à celle nécessaire pour transformer, selon le cas, la fonction alcool en aldéhyde ou cétone.

La température réactionnelle à adopter varie selon la stabilité thermique des produits à préparer.

Conformément à l'invention, la température est choisie de préférence, dans une gamme de température allant de 45°C à 100°C, et encore plus préférentiellement entre 60°C et 80°C.

En fin de réaction qui dure de préférence, entre 30 minutes et 4 heures, on récupère l'éther aromatique porteur d'un groupe oxydé.

Puis, après refroidissement s'il y a lieu, on sépare la masse catalytique de la masse réactionnelle, par exemple par filtration.

On récupère l'aldéhyde ou la cétone aromatique obtenue, selon les techniques classiques de séparation, de préférence, par distillation ou cristallisation.

Comme mentionné précédemment, le procédé de l'invention est particulièrement bien adapté pour la préparation de la vanilline et de l'éthylvanilline.

Les exemples qui suivent, illustrent l'invention sans toutefois la limiter.
Par conversion et sélectivité, on entend :

$$\text{Taux de conversion} = \frac{\text{nombre de moles d'éther transformées}}{\text{nombre de moles d'éther introduites}} \text{ en \%}$$

$$\text{Sélectivité} = \frac{\text{nombre de moles d'éther hydroxyalkylé formées}}{\text{nombre de moles d'éther transformées}} \text{ en \%}$$

Exemple 1:

Dans un réacteur tricol de 50 ml muni d'une agitation mécanique, d'un réfrigérant ascendant et d'un système de prélèvement d'échantillons, on charge 0,5 g d'une zéolithe H-mordénite de rapport molaire Si/Al = 18 et 12 ml d'une solution aqueuse de formol (37 % en poids, 160 mmol).

Le mélange réactionnel est chauffé à 65°C à l'aide d'un bain thermostaté et est soumis à une agitation de 500 tours/min.

Lorsque le systimé est en équilibre thermique, on introduit 1 ml de gaïacol (9 mmol).

Des prélèvements sont périodiquement efflectués et analysés par chromatographie liquide à haute performance.

Après 1 heure et 2 heures de réaction, on obtient les performances suivantes :

| 1 heure | 2 heures |
|---|---|
| Conversion/gaïacol = 18 %<br>Sélectivité/p-vanillole = 68 %<br>Sélectivité/o-vanillole = 14 % | Conversio/gaïacol = 32 %<br>Sélectivité/p- vanillole = 62 %<br>Sélectivité/o-vanillole = 13 % |

Exemples 2 à 5 :

Par utilisation du même catalyseur, l'exemple est répété en faisant varier, soit la température, soit la quantité de catalyseur.

Le milieu réactionnel est analysé après différentes durées et les résultats sont consignés dans le tableau suivant :

Tableau I

| Ex. | Température (°C) | Quantité de catalyseur (g) | Conversion/ gaïacol (%) | Sélectivité/p-vanillole (%) | Sélectivité/o-vanillole (%) | Durée (min) |
|---|---|---|---|---|---|---|
| 2 | 85 | 0,5 | 32<br>49<br>73 | 48<br>58<br>49 | 10<br>11<br>11 | 30<br>60<br>120 |
| 3 | 85 | 0,25 | 23<br>32<br>47 | 34<br>38<br>43 | 7<br>8<br>10 | 30<br>60<br>120 |

Tableau I (suite)

| Ex. | Température (°C) | Quantité de catalyseur (g) | Conversion/ gaïacol (%) | Sélectivité/p-vanillole (%) | Sélectivité/o-vanillole (%) | Durée (min) |
|---|---|---|---|---|---|---|
| 4 | 65 | 0,75 | 19<br>42<br>50 | 59<br>56<br>55 | 11<br>13<br>11 | 30<br>60<br>120 |
| 5 | 50 | 0,5 | 9 | 53 | 9 | 120 |

<u>Exemples 6 à 11</u>

Le mode opératoire décrit dans l'exemple 1 est reproduit par utilisation de 0,25 g de zéolithes de structure et de rapport molaire Si/Al variable et à une température de 85°C. Les résultats sont consignés dans le tableau suivant :

Tableau (II)

| Ex. | Zéolithe | Rapport molaire Si/Al | Durée (min) | Conversion/ gaïacol (%) | Sélectivité/p-vanillole (%) | Sélectivité/o-vanillole (%) |
|---|---|---|---|---|---|---|
| 6 | H-mordénite | 10 | 120 | 12 | 24 | 5 |
| 7 | H-mordénite | 15 | 30<br>60<br>120 | 12<br>19<br>28 | 41<br>47<br>53 | 8<br>9<br>11 |
| 8 | H-mordénite | 49 | 30<br>60<br>120 | 10<br>19<br>34 | 36<br>37<br>29 | 10<br>9<br>8 |
| 9 | H-mordénite | 108 | 30<br>60<br>120 | 53<br>74<br>86 | 28<br>25<br>22 | 5<br>5<br>7 |
| 10 | H-β | 12,5 | 30<br>60<br>120 | 22<br>30<br>44 | 15<br>16<br>23 | 7<br>8<br>8 |
| 11 | H-β | 43 | 30<br>60<br>120 | 37<br>50<br>58 | 35<br>30<br>25 | 9<br>7<br>7 |

<u>Exemples 12 à 15</u>

L'exemple 1 est reproduit par utilisation de 0,25 g de zéolithe H-mordénite de rapport molaire Si/Al = 100 à 85°C en faisant varier la quantité de formol et la concentration.

Le milieu réactionnel est analysé après différentes durées et les résultats sont consignés dans le tableau suivant :

Tableau (III)

| Ex. | HCHO (mmol) | $C_{gaïacol}$ (mol/l) | Durée (min) | Conversion/gaïacol (%) | Sélectivité/p-vanillole (%) | Sélectivité/o-vanillole (%) |
|---|---|---|---|---|---|---|
| 12 | 80 | 0,7 | 30<br>60<br>120 | 54<br>74<br>77 | 9<br>6<br>7 | 6<br>4<br>5 |
| 13 | 160 | 0,7 | 30<br>60 | 42<br>59 | 28<br>29 | 7<br>7 |

Tableau (III) (suite)

| Ex. | HCHO (mmol) | $C_{gaïacol}$ (mol/l) | Durée (min) | Conversion/gaïacol (%) | Sélectivité/p-vanillole (%) | Sélectivité/o-vanillole (%) |
|---|---|---|---|---|---|---|
| | | | 120 | 68 | 35 | 9 |
| 14 | 240 | 0,45 | 30<br>60<br>120 | 32<br>54<br>67 | 38<br>36<br>31 | 9<br>8<br>7 |
| 15 | 80 | 1,2 | 30<br>60<br>120 | 43<br>62<br>76 | 17<br>17<br>22 | 4<br>6<br>3 |

Exemple 16

On reproduit l'exemple 1 mais en mettant en jeu l'anisole à la place du gaïacol.
On charge 0,75 g de zéolithe H-mordénite de rapport molaire Si/Al = 18, 1 ml d'anisole et 12 ml d'une solution aqueuse de formol (37 % en poids, 160 mmol).
Le mélange réactionnel est chauffé, sous agitation, à 65°C.
Après 4 heures de réaction, on obtient les performances suivantes :

- Conversion/anisole = 89 %
- Sélectivité/p-hydroxyméthylanisole = 45 %
- Sélectivité/o-hydroxyméthylanisole = 5 %

Exemple 17

Cet exemple illustre la préparation de la vanilline à partir d'alcool p-vanillique obtenu selon l'exemple 1.
Dans un réacteur en vere équipé de chicanes, on introduit 31 g d'alcool p-vanillique obtenu selon l'exemple 1, 250 g d'eau et 0,2 mol de soude apportée sous forme d'une solution aqueuse 4N.
On ajoute 3 g d'un catalyseur à base de platine 2 % déposé sur carbone et 10 mg de sulfate de bismuth.
On purge le réacteur à l'oxygène et on le met ensuite en relation avec une réserve d'oxygène en établissant une légère pression.
On chauffe le mélange réactionnel à 45 °C et on agite à 1000 tours/mn.
On maintient le milieu sous agitation à cette température jusqu'à ce que le volume d'oxygène corresponde à la quantité théorique pour transformer l'alcool en aldéhyde.
On arrête l'opération et l'on purge le réacteur à l'azote.
On acidifie le milieu avec une solution aqueuse d'acide chlorhydrique jusqu'à pH de 6.
On extrait la vanilline obtenue par 4 fois 100 ml de toluène.
On détermine par dosage par chromatographie liquide haute performance un taux de conversion de 100 % et un rendement en vanilline (nombre de moles de vanilline formées/nombre de moles d'alcool p-vanillique engagées) de 92 %.

## Revendications

1. Procédé de préparation d'un éther aromatique carbocyclique hydroxyalkylé qui consiste à faire réagir ledit éther aromatique avec un composé carbonylé, en présence d'un catalyseur, ledit procédé étant **caractérisé par le fait que** l'on conduit la réaction d'hydroxyalkylation, à une température inférieure ou égale à 100°C, en présence d'une quantité efficace d'une zéolithe.

2. Procédé selon la revendication 1 **caractérisé par le fait que** l'éther aromatique carbocydique répond à la formule générale (I) :

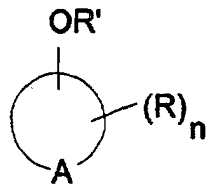

(I)

dans laquelle :

- A symbolise le reste d'un cycle formant tout ou partie d'un système carbocyclique aromatique, monocyclique ou polycyclique, système comprenant au moins un groupe OR' : ledit reste cyclique pouvant porter un ou plusieurs substituants,
- R représente un ou plusieurs substituants, identiques ou différents,
- R' représente un radical hydrocarboné ayant de 1 à 24 atomes de carbone, qui peut être un radical aliphatique acyclique saturé ou insaturé, linéaire ou ramifié ; un radical cycloaliphatique saturé, insaturé ou aromatique, monocyclique ou polycyclique ; un radical aliphatique saturé ou insaturé, linéaire ou ramifié, porteur d'un substituant cyclique,
- R' et R peuvent former un cycle comprenant éventuellement un autre hétéroatome,
- n est un nombre inférieur ou égal à 4.

3. Procédé selon l'une des revendications 1 et 2 **caractérisé par le fait que** l'éther aromatique carbocyclique répond à la formule générale (I) dans laquelle R' représente:

- un radical aliphatique acyclique, saturé ou insaturé, linéaire ou ramifié, de préférence un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome, un groupe fonctionnel et/ou porteuse d'un substituant,
- un radical aliphatique acyclique, saturé ou insaturé, linéaire ou ramifié porteur d'un substituant cyclique éventuellement substitué : ledit radical acyclique pouvant être relié au cycle par un lien valentiel, un hétéroatome ou un groupe fonctionnel,
- un radical carbocyclique saturé ou comprenant 1 ou 2 insaturations dans le cycle, ayant généralement de 3 à 8 atomes de carbone, de préférence, 6 atomes de carbone dans le cycle ; ledit cycle pouvant être substitué.
- un radical carbocyclique aromatique, de préférence monocyclique ayant généralement au moins 4 atomes de carbone, de préférence, 6 atomes de carbone dans le cycle ; ledit cycle pouvant être substitué.

4. Procédé selon la revendication 1 **caractérisé par le fait que** l'éther aromatique carbocyclique répond à la formule générale (I) dans laquelle R' représente un radical alkyle linéaire ou ramifié ayant de 1 à 4 atomes de carbone, de préférence un radical méthyle ou un radical phényle.

5. Procédé selon l'une des revendications 1 à 4 **caractérisé par le fait que** l'éther aromatique carbocyclique répond à la formule générale (I) dans laquelle le reste A représente le reste d'un composé carbocyclique aromatique, monocyclique ayant au moins 4 atomes de carbone et de préférence 6 atomes de carbone ou le reste d'un composé carbocyclique polycyclique, de préférence un reste naphtalénique : le reste A pouvant porter un ou plusieurs substituants sur le noyau aromatique.

6. Procédé selon l'une des revendications 1 à 5 **caractérisé par le fait que** l'éther aromatique carbocyclique répond à la formule (Ia) :

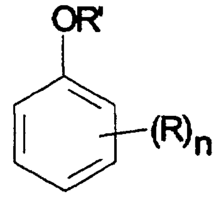

(Ia)

dans laquelle :

- n est un nombre inférieur ou égal à 4, de préférence égal à 0, 1 ou 2,
- le radical R' représente un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle ou phényle,
- le ou les radicaux R représentent l'un des atomes ou groupes suivants :

  . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  . un radical alcényle linéaire ou ramifié ayant de 2 à 6 atomes de carbone, de préférence, de 2 à 4 atomes de carbone, tel que vinyle, allyle,
  . un radical cyclohexyle ou benzyle,
  . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  . un groupe acyle ayant de 2 à 6 atomes de carbone,
  . un radical de formule :

$$-R_1-OH$$

$$-R_1-COOR_2$$

$$-R_1-CHO$$

$$-R_1-NO_2$$

$$-R_1-CN$$

$$-R_1-N-(R_2)_2$$

$$-R_1-CO-N-(R_2)_2$$

$$-R_1-X$$

$$-R_1-CF_3$$

dans lesdites formules, $R_1$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; les radicaus $R_2$, identiques ou différents, représentent un atome

d'hydrogène ou un radical alkyle linéaire ou ramifié ayant de 1 à 6 atomes de carbone ; X symbolise un atome d'halogène, de préférence un atome de chlore, de brome ou de fluor.

- les radicaux R' et R et les 2 atomes successifs du cycle benzénique peuvent former entre eux, un cycle ayant de 5 à 7 atomes, comprenant éventuellement un autre hétéroatome.

**7.** Procédé selon la revendication 6 **caractérisé par le fait que** l'éther aromatique carbocyclique répond à la formule (Ia) dans laquelle n est supérieur ou égal à 1, les radicaux R' et R et les 2 atomes successifs du cycle benzénique peuvent être liés entre eux par un radical alkylène, alcénylène ou alcénylidène ayant de 2 à 4 atomes de carbone pour former un hétérocycle saturé, insaturé ou aromatique ayant de 5 à 7 atomes de carbone dans lequel un ou plusieurs atomes de carbone peuvent être remplacés par un hétéroatome, de préférence l'oxygène : les radicaux OR' et R formant de préférence un radical méthylène dioxy ou éthylène dioxy.

**8.** Procédé selon l'une des revendications 6 et 7 **caractérisé par le fait que** l'éther aromatique carbocyclique est un éther aromatique répondant à la formule (Ia) dans laquelle n est égal à 1, le radical R' représente un radical alkyle ayant de 1 à 4 atomes de carbone et R représente un radical alkoxy ayant de 1 à 4 atomes de carbone ou un groupe hydroxyle.

**9.** Procédé selon la revendication 1 et 2 **caractérisé par le fait que** l'éther aromatique carbocyclique est l'anisole, le phénétole, le gaïacol, le guétol, le vératrole, le 1,2-méthylènedioxybenzène, le 2-méthoxynaphtalène.

**10.** Procédé selon l'une des revendications 1 à 9 **caractérisé par le fait que** le composé carbonylé répond à la formule (II) :

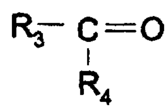

**(II)**

dans ladite formule (II) :

- $R_3$ et $R_4$, identiques ou différents, représentent :

  . un atome d'hydrogène,
  . un radical alkyle, linéaire ou ramifié, ayant de 1 à 6 atomes de carbone,
  . un groupe alcényle, linéaire ou ramifié, ayant de 2 à 6 atomes de carbone,
  . un groupe phényle,
  . un groupe électro-attracteur.

**11.** Procédé selon la revendication 10 **caractérisé par le fait que** le composé carbonylé répond à la formule (II) dans laquelle le groupe électro-attracteur est choisi parmi :

- un groupe -CHO,
- un groupe $-COOR_5$ dans lequel $R_5$ représente un atome d'hydrogène ou un radical alkyle linéaire ou ramifié ayant de 1 à 6 atomes de carbone,
- un groupe $-CX_2H$ dans lequel X représente un atome d'halogène, de préférence, un atome de chlore,
- un groupe $-CX_3$ dans lequel X représente un atome d'halogène, de préférence, un atome de chlore.

**12.** Procédé selon la revendication 10 et 11 **caractérisé par le fait que** le composé carbonylé est choisi parmi :

- le formaldéhyde ou un générateur de formaldéhyde tel que, par exemple, le trioxane ou le paraformaldéhyde utilisé sous la forme de polyformaldéhydes linéaires de degré de polymérisation indifférent, ayant de préférence un nombre de motifs ($CH_2O$) compris entre 8 et 100 motifs,
- l'acide glyoxylique,
- le chloral,
- la dichloroacétone,
- l'acétaldéhyde,

- le propionaldéhyde,
- le dichloroacétaldéhyde,
- le trichloroacétaldéhyde,
- le pyruvate de méthyle,
- le pyruvate d'éthyle.

**13.** Procédé selon l'une des revendications 1 à 12 **caractérisé par le fait que** le composé carbonylé est le formaldéhyde ou un générateur de formaldéhyde.

**14.** Procédé selon l'une des revendications 1 à 13 **caractérisé par le fait que** la zéolithe est une zéolithe naturelle ou synthétique.

**15.** Procédé selon la revendication 15 **caractérisé par le fait que** la zéolithe est une zéolithe naturelle choisie parmi ; la chabazite, la clinoptilolite, l'érionite, la mordénite, la phillipsite, l'offrétite.

**16.** Procédé selon la revendication 15 **caractérisé par le fait que** la zéolithe est une zéolithe synthétique choisie parmi :

- les zéolithes synthétiques à réseau monodimensionnel telles que la zéolithe ZSM-4, la zéolithe L, la zéolithe ZSM-12, la zéolithe ZSM-22, la zéolithe ZSM-23, la zéolithe ZSM-48.
- les zéolithes à réseau bidimensionnel telles que la mordénite, la ferrierite,
- les zéolithes à réseau tridimensionnel telles que la zéolithe β, la zéolithe Y, la zéolithe X, la zéolithe ZSM-5, la zéolithe ZSM-11, l'offrétite.

**17.** Procédé selon la revendication 16 **caractérisé par le fait que** la zéolithe est une zéolithe H-β ou une H-mordénite.

**18.** Procédé selon l'une des revendications 1 à 17 **caractérisé par le fait que** l'on effectue la réaction en milieu aqueux ou en présence d'un solvant organique choisi parmi les hydrocarbures aliphatiques et/ou aromatiques éventuellement halogénés, de préférence chlorés, les éther-oxydes aliphatiques, cycloaliphatiques ou aromatiques, les solvants aprotiques polaires, de préférence les composés nitrés, les nitriles aliphatiques ou aromatiques, la tétraméthylènesulfone, le diméthylsulfoxyde.

**19.** Procédé selon l'une des revendications 1 à 18 **caractérisé par le fait que** le rapport entre le nombre de moles de composé carbonylé et le nombre de moles d'éther aromatique varie entre 1 et 50 ; de préférence, de 1 à 25 lors de la mise en oeuvre de zéolithes naturelles ou synthétiques telles que les zéolithes L, X, Y, les mordénites de rapport molaire Si/Al de 5 à 12, la ferrierite et l'offrétite ; de préférence, de 8 à 25 dans le cas des zéolithes synthétique, telles que la zéolithe ZSM-4, la zéolithe ZSM-5, la zéolithe ZSM-11, la zéolithe ZSM-12, la zéolithe ZSM-22, la zéolithe ZSM-23, la zéolithe ZSM-48, la zéolithe L, la zéolithe β et les mordénites de rapport molaire Si/Al de 12 à 150.

**20.** Procédé selon l'une des revendications 1 à 19 **caractérisé par le fait que** la quantité de catalyseur représente en poids par rapport à l'éther aromatique engagé, de 5 à 80 %, de préférence, de 10 à 50 %.

**21.** Procédé selon l'une des revendications 1 à 20 **caractérisé par le fait que** la température à laquelle est mise en oeuvre la réaction d'hydroxyalkylation est inférieure ou égale à 100°C, de préférence entre 20°C et 100°C et encore plus préférentiellement entre 40°C et 90°C.

**22.** Procédé selon l'une des revendications 1 à 21 **caractérisé par le fait que** l'on charge le catalyseur, le composé carbonylé de formule (II), éventuellement un solvant organique puis l'on introduit l'éther aromatique carbocyclique en continu ou par fractions puis l'on porte le mélange réactionnel à la température souhaitée ou que l'on conduit la réaction en continu, dans un réacteur tubulaire comportant le catalyseur solide disposé en lit fixe.

**23.** Procédé selon la revendication 22 **caractérisé par le fait que** l'on récupère en fin de réaction, une phase liquide comprenant l'éther aromatique hydroxyalkylé qui est extrait dans un solvant organique puis la phase organique obtenue est soumise soit directement à une oxydation, soit à une contre-extraction par une solution aqueuse basique afin d'obtenir en phase aqueuse comprenant le produit sous forme salifiée qui est ensuite oxydé.

**24.** Procédé selon la revendication 23 **caractérisé par le fait que** le solvant d'extraction est choisi les hydrocarbures

aliphatiques ou aromatiques halogénés comme le chlorométhane, le dichlorométhane, le 1,2-dichloroéthane, le chlorobenzène, les dichlorobenzènes, les trichlorobenzènes ; les esters comme les acétates de butyle, d'isopropyle, d'amyle, de cyclohexyle ; les cétones telles que par exemple l'acétone, la méthyléthylcétone, la méthylisobutylcétone.

**25.** Procédé selon l'une des revendications 22 à 24 **caractérisé par le fait que** la solution aqueuse de contre-extraction est une solution aqueuse d'hydroxyde de sodium ou de potassium : la proportion de base minérale à utiliser est comprise entre 0,5 et 3 moles d'hydroxyde de sodium ou de potassium par rapport à l'éther aromatique hydroxyalkylé à oxyder.

**26.** Procédé d'oxydation d'un éther aromatique carbocyclique hydroxyalkylé **caractérisé par le fait qu'**il consiste :

- à préparer ledit composé selon le procédé décrit dans l'une des revendications 1 à 25, en faisant réagir un éther aromatique avec un composé carbonylé, à une température inférieure ou égale à 100°C, en présence d'une quantité efficace d'une zéolithe,
- à récupérer ledit éther aromatique hydroxyalkylé, en phase liquide, puis à l'oxyder à l'aide d'oxygène moléculaire ou un gaz en contenant, en présence d'un catalyseur à base d'un métal M choisi parmi les métaux du groupe 8 de la classification périodique comprenant éventuellement, à titre d'activateurs des métaux tels que le cadmium, le cérium, le bismuth, le plomb, l'argent, le tellure ou l'étain.

**27.** Procédé selon la revendication 26 **caractérisé par le fait que** le catalyseur est à base de nickel, de ruthénium, de rhodium, de palladium, d'osmium, d'iridium, de platine ou de leurs mélanges de préférence à base de platine et/ou de palladium.

**28.** Procédé selon l'une des revendications 26 et 27 **caractérisé par le fait que** le catalyseur au platine est apporté sous forme de noir de platine, d'oxyde de platine, ou de métal noble lui-même déposé sur des supports divers tels que le noir de carbone, le carbonate de calcium, les alumines et silices activées ou des matériaux équivalents, de préférence le noir de carbone.

**29.** Procédé selon l'une des revendications 26 à 28 **caractérisé par le fait que** la quantité de catalyseur à mettre en oeuvre, exprimée en poids de métal M par rapport à celui de l'éther aromatique hydroxyalkylé, peut varier de 0,01 à 4 % et, de préférence, de 0,04 à 2 %.

**30.** Procédé selon l'une des revendication 26 à 29 **caractérisé par le fait que** l'activateur est un dérivé organique ou inorganique du bismuth pris dans le groupe formé par : les oxydes de bismuth ; les hydroxydes de bismuth ; les sels de bismuth ou de bismuthyle d'hydracides minéraux, de préférence les chlorure, bromure, iodure, sulfure, sélénure, tellure ; les sels de bismuth ou de bismuthyle d'oxyacides minéraux, de préférence les sulfite, sulfate, nitrite, nitrate, phosphite, phosphate, pyrophosphate, carbonate, perchlorate, antimoniate, arséniate, sélénite, séléniate ; les sels de bismuth ou de bismuthyle d'acides organiques aliphatiques ou aromatiques, de préférence les acétate, propionate, salicylate, benzoate, oxalate, tartrate, lactate, citrate ; les phénates de bismuth ou de bismuthyle, de préférence les gallate et pyrogallate.

**31.** Procédé selon la revendication 30 **caractérisé par le fait que** le dérivé du bismuth est pris dans le groupe formé par : les oxydes de bismuth $Bi_2O_3$ et $Bi_2O_4$ ; l'hydroxyde de bismuth $Bi(OH)_3$ ; le chlorure de bismuth $BiCl_3$ ; le bromure de bismuth $BiBr_3$ ; l'iodure de bismuth $BiI_3$ ; le sulfate neutre de bismuth $Bi_2(SO_4)_3$ ; le nitrate neutre de bismuth $Bi(NO_3)_3$, $5H_2O$ ; le nitrate de bismuthyle $BiO(NO_3)$ ; le carbonate de bismuthyle $(BiO)_2CO_3$, 0,5 $H_2O$ ; l'acétate de bismuth $Bi(C_2H_3O_2)_3$ ; le salicylate de bismuthyle $C_6H_4CO_2(BiO)OH$.

**32.** Procédé selon l'une des revendications 26 à 31 **caractérisé par le fait que** la quantité d'activateur est choisie de manière qu'elle apporte dans la milieu : d'une part, au moins 0,1 % en poids de métal activateur par rapport au poids du métal M engagé, et d'autre part de 10 à 900 ppm en poids de métal M par rapport à l'éther aromatique hydroxyalkylé.

**33.** Procédé selon l'une des revendications 26 à 32 **caractérisé par le fait que** la réaction d'oxydation est conduite dans une gamme de température allant de 45°C à 100°C, et encore plus préférentiellement entre 60°C et 80°C.

**34.** Procédé de préparation de la vanilline par oxydation de l'alcool 3-méthoxy-4-hydroxybenzylique **caractérisé par le fait que** l'on prépare l'alcool 3-méthoxy-4-hydroxybenzylique par hydroxyméthylation du gaïacol effectuée selon

le procédé décrit dans l'une des revendications 1 à 25, à une température inférieure ou égale à 100°C, en présence d'une quantité efficace d'une zéolithe.

**35.** Procédé selon la revendication 34 **caractérisé par le fait que** l'on récupère l'alcool 3-méthoxy-4-hydroxybenzylique en phase liquide, puis que l'on effectue son oxydation selon le procédé décrit dans l'une des revendications 26 à 33, à l'aide d'oxygène moléculaire ou un gaz en contenant, en présence d'un catalyseur à base d'un métal M choisi parmi les métaux du groupe 8 de la classification périodique comprenant éventuellement, à titre d'activateurs des métaux tels que le cadmium, le cérium, le bismuth, le plomb, l'argent, le tellure ou l'étain.

**36.** Procédé de préparation de l'éthylvanilline par oxydation de l'alcool 3-éthoxy-4-hydroxybenzylique **caractérisé par le fait que** l'on prépare l'alcool 3-éthoxy-4-hydroxybenzylique par hydroxyméthylation du guétol effectuée selon le procédé décrit dans l'une des revendications 1 à 25, à une température inférieure ou égale à 100°C, en présence d'une quantité efficace d'une zéolithe.

**37.** Procédé selon la revendication 36 **caractérisé par le fait que** l'on récupère l'alcool 3-éthoxy-4-hydroxybenzylique en phase liquide puis que l'on effectue son oxydation selon le procédé décrit dans l'une des revendications 26 à 33, à l'aide d'oxygène moléculaire ou un gaz en contenant, en présence d'un catalyseur à base d'un métal M choisi parmi les métaux du groupe 8 de la classification périodique comprenant éventuellement, à titre d'activateurs des métaux tels que le cadmium, le cérium, le bismuth, le plomb, l'argent, le tellure ou l'étain.

## Patentansprüche

**1.** Verfahren zur Herstellung eines hydroxyalkylierten carbocyclischen aromatischen Ethers durch Reaktion des aromatischen Ethers mit einer Carbonylverbindung in Gegenwart eines Katalysators, **dadurch gekennzeichnet ist, daß** man die Hydroxyalkylierungsreaktion bei einer Temperatur kleiner oder gleich 100 °C in Gegenwart einer wirksamen Menge eines Zeolithen durchführt.

**2.** Verfahren nach Anspruch 1, **dadurch gekennzeichnet, daß** der carbocyclische aromatische Ether der allgemeinen Formel (I) entspricht:

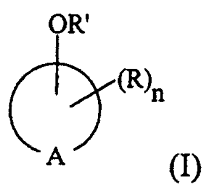

(I)

in der:

- A den Rest eines Ringes darstellt, der vollständig oder teilweise ein polycyclisches oder monocyclisches, carbocyclisches aromatisches System bildet, das mindestens eine Gruppe OR' umfaßt, wobei der cyclische Rest Träger eines oder mehrerer Substituenten sein kann,
- R einen oder mehrere, gleiche oder verschiedene Substituenten darstellt,
- R' einen Kohlenwasserstoffrest mit 1 bis 24 Kohlenstoffatomen darstellt, der ein linearer oder verzweigter, gesättigter oder ungesättigter, acyclischer aliphatischer Rest, ein monocyclischer oder polycyclischer, gesättigter, ungesättigter oder aromatischer cycloaliphatischer Rest oder ein linearer oder verzweigter, gesättigter oder ungesättigter aliphatischer Rest, der Träger eines cyclischen Substituenten ist, sein kann,
- R' und R einen Ring bilden können, der gegebenenfalls ein anderes Heteroatom enthält,
- n eine Zahl kleiner oder gleich 4 ist.

**3.** Verfahren nach Anspruch 1 oder 2, **dadurch gekennzeichnet, daß** der carbocyclische aromatische Ether der allgemeinen Formel (I) entspricht, in der R' darstellt:

- einen linearen oder verzweigten, gesättigten oder ungesättigten, acyclischen aliphatische Rest, vorzugsweise einen linearen oder verzweigten Alkylrest mit 1 bis 12 Kohlenstoffatomen, vorzugsweise 1 bis 6 Kohlenstoffatomen, wobei die Kohlenwasserstoffkette gegebenenfalls durch ein Heteroatom oder eine funktionelle Gruppe unterbrochen sein kann und/oder Träger eines Substituenten sein kann,
- einen linearen oder verzweigten, gesättigten oder ungesättigten acyclischen aliphatischen Rest, der Träger eines gegebenenfalls substituierten cyclischen Substituenten ist, wobei der acyclische Rest über eine Valenzbindung, ein Heteroatom oder eine fünktionelle Gruppe mit dem Ring verbunden sein kann,
- einen gesättigten oder 1 oder 2 ungesättigte Stellen in dem Ring aufweisender carbocyclischer Rest mit im allgemeinen 3 bis 8 Kohlenstoffatomen, vorzugsweise 6 Kohlenstoffatomen, im Ring, wobei der Ring substituiert sein kann,
- einen vorzugsweise monocyclischen, aromatischen carbocyclischen Rest mit im allgemeinen mindestens 4 Kohlenstoffatomen, vorzugsweise 6 Kohlenstoffatomen, im Ring, wobei der Ring substituiert sein kann.

4. Verfahren nach Anspruch 1, **dadurch gekennzeichnet, daß** der carbocyclische aromatische Ether der allgemeinen Formel (I) entspricht, in der R' einen linearen oder verzeigten Alkylrest mit 1 bis 4 Kohlenstoffatomen, vorzugsweise einen Methylrest oder einen Phenylrest, darstellt.

5. Verfahren nach einem der Ansprüche 1 bis 4, **dadurch gekennzeichnet, daß** der carbocyclische aromatische Ether der allgemeinen Formel (I) entspricht, in der der Rest A den Rest einer monocyclischen aromatischen carbocyclischen Verbindung mit mindestens 4 Kohlenstoffatomen, vorzugsweise 6 Kohlenstoffatomen, oder den Rest einer polycyclischen carbocyclischen Verbindung, vorzugsweise einen Naphthalinrest, darstellt, wobei der Rest A Träger eines oder mehrerer Substituenten an dem aromatischen Kern sein kann.

6. Verfahren nach einem der Ansprüche 1 bis 5, **dadurch gekennzeichnet, daß** der carbocyclische aromatische Ether der Formel (Ia) entspricht:

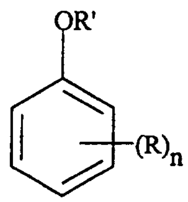

(Ia)

in der:

- n eine Zahl kleiner oder gleich 4, vorzugsweise gleich 0,1 oder 2, ist,
- der Rest R' einen linearen oder verzweigten Alkylrest mit 1 bis 6 Kohlenstoffatomen, vorzugsweise 1 bis 4 Kohlenstoffatomen, darstellt, wie Methyl, Ethyl, Propyl, Isopropyl, Butyl, Isobutyl, sec.-Butyl, tert.-Butyl oder Phenyl,
- der oder die Rest(e) R eines oder einer der folgenden Atome oder Gruppen entspricht:
  - einem linearen oder verzweigten Alkylrest mit 1 bis 6 Kohlenstoffatomen, vorzugsweise 1 bis 4 Kohlenstoffatomen, wie Methyl, Ethyl, Propyl, Isopropyl, Butyl, Isobutyl, sec.-Butyl, tert.-Butyl,
  - einem linearen oder verzweigten Alkenylrest mit 2 bis 6 Kohlenstoffatomen, vorzugsweise 2 bis 4 Kohlenstoffatomen, wie Vinyl oder Allyl,
  - einem Cyclohexyl- oder Benzylrest,
  - einem linearen oder verzweigten Alkoxyrest mit 1 bis 6 Kohlenstoffatomen, vorzugsweise 1 bis 4 Kohlenstoffatomen, wie Methoxy-, Ethoxy-, Propoxy-, Isopropoxy- oder Butoxyresten,
  - einer acyclischen Gruppe mit 2 bis 6 Kohlenstoffatomen,

- einem Rest der Formel:
  - $- R_1-OH$,
  - $- R_1-COOR_2$,
  - $- R_1-CHO$,
  - $- R_1-NO_2$,
  - $- R_1-CN$,
  - $- R_1-N-(R_2)_2$,
  - $- R_1-CO-N-(R_2)_2$,
  - $- R_1-X$,
  - $- R_1-CF_3$,

  wobei in den Formeln $R_1$ eine Valenzbindung oder einen gesättigten oder ungesättigten, linearen oder verzweigten, zweibindigen (divalenten) Kohlenwasserstoffrest mit 1 bis 6 Kohlenstoffatomen darstellt, wie z. B. Methylen, Ethylen, Propylen, Isopropylen oder Isopropyliden, die Reste $R_2$, identisch oder verschieden, ein Wasserstoffatom oder einen linearen oder verzweigten Alkylrest mit 1 bis 6 Kohlenstoffatomen darstellen, X ein Halogenatom, vorzugsweise ein Chlor-, Brom- oder Fluoratom, darstellt,

- die Reste R' und R und die zwei aufeinander folgenden Atome des Benzolringes untereinander einen Ring mit 5 bis 7 Atomen, der gegebenenfalls andere Heteroatome enthält, bilden können.

7. Verfahren nach Anspruch 6, **dadurch gekennzeichnet, daß** der carbocyclische aromatische Ether der Formel (Ia) entspricht, in der n größer oder gleich 1 ist, die Reste R' und R und die zwei folgenden Atome des Benzolringes untereinander durch einen Alkenyl-, Alkenylen- oder Alkenylidenrest mit 2 bis 4 Kohlenstoffatomen verbunden sein können, um einen gesättigten, ungesättigten oder aromatischen Heterocyclus mit 5 bis 7 Kohlenstoffatomen zu bilden, in dem ein oder mehrere Kohlenstoffatom(e) durch ein Heteroatom, vorzugsweise ein Sauerstoffatom, ersetzt sein können, wobei die Reste OR' und R vorzugsweise einen Methylendioxy- oder Ethylendioxyrest bilden.

8. Verfahren nach Anspruch 6 oder 7, **dadurch gekennzeichnet, daß** der carbocyclische aromatische Ether ein aromatischer Ether der Formel (Ia) ist, in der n gleich 1 ist, der Rest R' einen Alkylrest mit 1 bis 4 Kohlenstoffatomen darstellt und R einen Alkoxyrest mit 1 bis 4 Kohlenstoffatomen oder eine Hydroxylgruppe darstellt.

9. Verfahren nach Anspruch 1 oder 2, **dadurch gekennzeichnet, daß** der carbocyclische aromatische Ether Anisol, Phenetol, Guajakol, Guetol, Veratrol, 1,2-Methylendioxybenzol oder 2-Methoxynaphthalin ist.

10. Verfahren nach einem der Ansprüche 1 bis 9, **dadurch gekennzeichnet, daß** die Carbonylverbindung der Formel (II) entspricht:

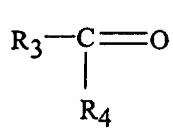

(II)

wobei in der Formel (II):

- $R_3$ und $R_4$, identisch oder verschieden, darstellen:
  - ein Wasserstoffatom,

- einen linearen oder verzweigten Alkylrest mit 1 bis 6 Kohlenstoffatomen,
- eine lineare oder verzweigten Alkenylgruppe mit 2 bis 6 Kohlenstoffatomen,
- eine Phenylgruppe,
- eine elektronenziehende Gruppe.

**11.** Verfahren nach Anspruch 10, **dadurch gekennzeichnet, daß** die Carbonylverbindung der Formel (II) entspricht, in der die elektronenziehende Gruppe ausgewählt ist aus:

- einer -CHO-Gruppe,
- einer $-COOR_5$-Gruppe, in der $R_5$ ein Wasserstoffatom oder einen linearen oder verzweigten Alkylrest mit 1 bis 6 Kohlenstoffatomen darstellt,
- einer $-CX_2H$-Gruppe, in der X ein Halogenatom, vorzugsweise ein Chloratom, darstellt,
- einer $-CX_3$-Gruppe, in der X ein Halogenatom, vorzugsweise ein Chloratom, darstellt.

**12.** Verfahren nach Anspruch 10 oder 11, **dadurch gekennzeichnet, daß** die Carbonylverbindung ausgewählt ist aus:

- Formaldehyd oder einem Formaldehyderzeuger, wie z. B. Trioxan oder Paraformaldehyd, das in Form von linearen Polyformaldehyden unterschiedlicher Polymerisationsgrade mit vorzugsweise einer Anzahl an $CH_2O$-Einheiten zwischen 8 und 100 Einheiten verwendet wird,
- Glyoxalsäure,
- Chloral,
- Dichloraceton,
- Acetaldehyd,
- Propionaldehyd,
- Dichloracetaldehyd,
- Trichloracetaldehyd,
- Methylpyruvat,
- Ethylpyruvat.

**13.** Verfahren nach einem der Ansprüche 1 bis 12, **dadurch gekennzeichnet, daß** die Carbonylverbindung Formaldehyd oder ein Formaldehyderzeuger ist.

**14.** Verfahren nach einem der Ansprüche 1 bis 13, **dadurch gekennzeichnet, daß** der Zeolith ein natürlicher oder synthetischer Zeolith ist.

**15.** Verfahren nach Anspruch 14, **dadurch gekennzeichnet, daß** der Zeolith ein natürlicher Zeolith ist, der ausgewählt ist aus Chabasit, Clinoptilolith, Erionit, Mordenit, Phillipsit oder Offretit.

**16.** Verfahren nach Anspruch 15, **dadurch gekennzeichnet, daß** der Zeolith ein synthetischer Zeolith ist, der ausgewählt ist aus:

- synthetischen Zeolithen mit eindimensionalen Gittern (Netzwerken), wie Zeolith ZSM-4, Zeolith L, Zeolith ZSM-12, Zeolith ZSM-22, Zeolith ZSM-23, Zeolith ZSM-48,

- Zeolithen mit zweidimensionalen Gittern (Netzwerken), wie Mordenit, Ferrierit,

- Zeolithen mit dreidimensionalen Gitter (Netzwerken), wie β-Zeolith, Zeolith Y, Zeolith X, Zeolith ZSM-5, Zeolith ZSM-11, Offretit.

**17.** Verfahren nach Anspruch 16, **dadurch gekennzeichnet, daß** der Zeolith ein H-β-Zeolith oder H-Mordenit ist.

**18.** Verfahren nach einem der Ansprüche 1 bis 17, **dadurch gekennzeichnet, daß** man die Reaktion in wäßrigem Milieu oder in Gegenwart eines organischen Lösemittels durchführt, das ausgewählt ist aus gegebenenfalls halogenierten, vorzugsweise chlorierten, aliphatischen und/oder aromatischen Kohlenwasserstoffen, aliphatischen, cycloaliphatischen oder aromatischen Etheroxiden, polar-aprotischen Lösemitteln, vorzugsweise nitrierten Verbindungen, aliphatischen oder aromatischen Nitrilen, Tetramethylensulfon oder Dimethylsulfoxid.

**19.** Verfahren nach einem der Ansprüche 1 bis 18, **dadurch gekennzeichnet, daß** das Verhältnis zwischen der Molzahl der Carbonylverbindung und der Molzahl des aromatischen Ether variiert zwischen 1 und 50, vorzugsweise 1 bis 25, bei der Verwendung von natürlichen oder synthetischen Zeolithen, wie Zeolithen L, X, Y und Mordeniten mit einem molaren Verhältnis Si/Al von 5 bis 12, Ferrierit und Offretit, und vorzugsweise von 8 bis 25 in dem Fall der synthetischen Zeolithe, wie Zeolith ZSM-4, Zeolith ZSM-5, Zeolith ZSM-11, Zeolith ZSM-12, Zeolith ZSM-22, Zeolith ZSM-23, Zeolith ZSM-48, Zeolith L, Zeolith β und den Mordeniten mit einem molaren Verhältnis Si/Al von 12 bis 150.

**20.** Verfahren nach einem der Ansprüche 1 bis 19, **dadurch gekennzeichnet, daß** die gewichtsbezogene Menge des Katalysators im Verhältnis zum verwendeten aromatischen Ether 5 bis 80 %, vorzugsweise 10 bis 50 %, entspricht.

**21.** Verfahren nach einem der Ansprüche 1 bis 20, **dadurch gekennzeichnet, daß** die Temperatur, bei der die Hydroxyalkylierungsreaktion durchgeführt wird, kleiner oder gleich 100 °C, vorzugsweise zwischen 20 °C und 100 °C, weiter bevorzugt zwischen 40 °C und 90 °C, beträgt.

**22.** Verfahren nach einem der Ansprüche 1 bis 21, **dadurch gekennzeichnet, daß** man den Katalysator, die Carbonylverbindung der Formel (II) und gegebenenfalls ein organische Lösemittel vorlegt und dann den carbocyclischen aromatischen Ether kontinuierlich oder portionsweise hinzugibt, dann das Reaktionsgemisch auf die gewünschte Temperatur bringt oder daß man die Reaktion kontinuierlich in einem röhrenförmigen Reaktor, der den festen Katalysator auf einem Festbett aufgetragen umfaßt, durchführt.

**23.** Verfahren nach Anspruch 22, **dadurch gekennzeichnet, daß** man am Ende der Reaktion eine flüssige Phase erhält, die den hydroxyalkylierten aromatischen Ether umfaßt, der in ein organisches Lösemittel extrahiert wird und die erhaltene organische Phase entweder sofort einer Oxidation oder einer Gegenextraktion durch eine basische wäßrige Lösung unterzogen wird, um in wäßriger Phase das Produkt in Salzform zu erhalten, das anschließend oxidiert wird.

**24.** Verfahren nach Anspruch 23, **dadurch gekennzeichnet, daß** das Extraktionslösemittel ausgewählt ist aus halogenierten, aliphatischen oder aromatischen Kohlenwasserstoffen, wie Chlormethan, Dichlormethan, 1,2-Dichlorethan, Chlorbenzol, Dichlorbenzolen, Trichlorbenzolen, Estern, wie Butyl-, Isopropyl-, Amyl-, Cyclohexylacetaten, Ketonen, wie z. B. Aceton, Methylethylketon, Methylisobutylketon.

**25.** Verfahren nach einem der Ansprüche 22 bis 24, **dadurch gekennzeichnet, daß** die wäßrige Lösung der Gegenextraktion eine wäßrige Natrium- oder Kaliumhydroxid-Lösung ist, wobei das Verhältnis an zu verwendender Mineralbase zum zu oxidierenden, hydroxyalkylierten aromatischen Ether zwischen 0,5 und 3 mol Kalium- oder Natriumhydroxid liegt.

**26.** Verfahren zur Oxidation eines hydroxyalkylierten carbocyclischen aromatischen Ethers, bestehend aus

- der Herstellung der Verbindung gemäß dem Verfahren, beschrieben in einem der Ansprüche 1 bis 25, durch Reaktion eines aromatischen Ethers mit einer Carbonylverbindung bei einer Temperatur kleiner oder gleich 100 °C in Gegenwart einer wirksamen Menge eines Zeolithen,

- dem Erhalt des hydroxyalkylierten aromatischen Ethers in flüssiger Phase, dann dessen Oxidation mit Hilfe von molekularem Sauerstoff oder einem sauerstoffhaltigem Gas in Gegenwart eines Katalysators auf Basis

eines Metalls M, das ausgewählt ist aus den Metallen der 8. Gruppe des Periodensystems, der gegebenenfalls als Beschleuniger (Aktivator) Metalle wie Cadmium, Cer, Bismut, Blei, Silber, Tellur oder Zinn umfaßt.

**27.** Verfahren nach Anspruch 26, **dadurch gekennzeichnet, daß** der Katalysator auf Nickel, Ruthenium, Rhodium, Palladium, Osmium, Iridium, Platin oder ihren Gemischen, vorzugsweise auf Platin und/oder Palladium, basiert.

**28.** Verfahren nach Anspruch 26 oder 27, **dadurch gekennzeichnet, daß** der Platinkatalysator in Form von Platinschwarz, Platinoxid oder als Edelmetall, das selber auf unterschiedlichen Trägern, wie Ruß, Calciumcarbonat, Aluminiumoxiden und Kieselerden oder ähnlichen Materialien, vorzugsweise Ruß, aufgetragen ist, eingebracht wird.

**29.** Verfahren nach einem der Ansprüche 26 bis 28, **dadurch gekennzeichnet, daß** die Menge des verwendeten Katalysators, ausgedrückt in Gew.-% des Metalls M im Verhältnis zu dem hydroxyalkylierten aromatischen Ether, von 0,01 bis 4 %, vorzugsweise 0,04 bis 2 %, variieren kann.

**30.** Verfahren nach einem der Ansprüche 26 bis 29, **dadurch gekennzeichnet, daß** der Beschleuniger ein organisches oder anorganisches Derivat des Bismuts ist, das ausgewählt ist aus der Gruppe von Bismutoxiden, Bismuthydroxiden, Bismut- oder Bismutoxidsalzen mineralischer Wasserstoffsäuren, vorzugsweise Chloriden, Bromiden, Iodiden, Sulfiden, Seleniden und Telluriden, Bismut- oder Bismutoxidsalzen mineralischer Oxysäuren, vorzugsweise Sulfiten, Sulfaten, Nitriten, Nitraten, Phosphiten, Phosphaten, Pyrophosphaten, Carbonaten, Perchloraten, Antimonaten, Arsenaten, Seleniten und Selenaten, Bismut- oder Bismutoxidsalzen aliphatischer oder aromatischer organischer Säuren, vorzugsweise Acetaten, Propionaten, Salicylaten, Benzoaten, Oxalaten, Tartraten, Lactaten und Citraten, Bismutoder Bismutoxidphenolaten, vorzugsweise Gallaten und Pyrogallaten.

**31.** Verfahren nach Anspruch 30, **dadurch gekennzeichnet, daß** das Bismutderivat ausgewählt ist aus der Gruppe von Bismutoxiden $Bi_2O_3$ und $Bi_2O_4$, Bismuthydroxid $Bi(OH)_3$, Bismutchlorid $BiCl_3$, Bismutbromid $BiBr_3$, Bismutiodid $BiI_3$, neutralem Bismutnitrat $Bi(NO_3)_3 \cdot 5\ H_2O$, Bismutoxidnitrat $BiO(NO_3)$, Bismutoxidcarbonat $(BiO)_2CO_3 \cdot 0{,}5\ H_2O$, Bismutacetat $Bi(C_2H_3O_2)_3$ oder Bismutoxidsalicylat $C_6H_4CO_2(BiO)OH$.

**32.** Verfahren nach einem der Ansprüche 26 bis 31, **dadurch gekennzeichnet, daß** die Menge an Beschleuniger derart ausgewählt ist, daß in dem Milieu einerseits mindestens 0,1 Gew.-% Beschleunigermetall im Gewichtsverhältnis zu dem verwendetem Metall M und andererseits 10 bis 900 Gew.ppm des Metalls M im Verhältnis zu dem hydroxyalkylierten aromatischen Ether bereitgestellt wird.

**33.** Verfahren nach einem der Ansprüche 26 bis 32, **dadurch gekennzeichnet, daß** die Oxidationsreaktion in einem Temperaturbereich von 45 °C bis 100 °C, weiter bevorzugt zwischen 60 °C und 80 °C, durchgeführt wird.

**34.** Verfahren zur Herstellung von Vanillin durch Oxidation von 3-Methoxy-4-hydroxybenzylalkohol, **dadurch gekennzeichnet, daß** man 3-Methoxy-4-hydroxybenzylalkohol durch Hydroxymethylierung von Gujakol gemäß dem Verfahren, beschrieben in den Ansprüchen 1 bis 25, bei einer Temperatur kleiner oder gleich 100 °C in Gegenwart einer wirksamen Menge eines Zeolithen herstellt.

**35.** Verfahren nach Anspruch 34, **dadurch gekennzeichnet, daß** man 3-Methoxy-4-hydroxybenzylalkohol in flüssiger Phase erhält, daß man dann seine Oxidation gemäß dem Verfahren, beschrieben in einem der Ansprüche 26 bis 33, mit Hilfe von molekularem Sauerstoff oder einem sauerstoffhaltigen Gas in Gegenwart eines Katalysators auf Basis eines Metalls M durchführt, das ausgewählt ist aus den Metallen der 8. Gruppe des Periodensystems, die gegebenenfalls Metalle wie Cadmium, Cer, Bismut, Blei, Silber, Tellur oder Zinn als Beschleuniger enthalten.

**36.** Verfahren zur Herstellung von Ethylvanillin durch Oxidation von 3-Ethoxy-4-hydroxybenzylalkohol, **dadurch gekennzeichnet, daß** man 3-Ethoxy-4-hydroxybenzylalkohol durch Hydroxymethylierung von Guetol gemäß dem Verfahren, beschrieben in den Ansprüchen 1 bis 25, bei einer Temperatur kleiner oder gleich 100 °C in Gegenwart einer wirksamen Menge eines Zeoliths herstellt.

**37.** Verfahren nach Anspruch 36, **dadurch gekennzeichnet, daß** man 3-Ethoxy-4-hydroxybenzylalkohol in flüssiger Phase erhält, daß man dann seine Oxidation gemäß dem Verfahren, beschrieben in einem der Ansprüche 26 bis 33, mit Hilfe von molekularem Sauerstoff oder einem sauerstoffhaltigen Gas in Gegenwart eines Katalysators auf Basis eines Metalls M durchführt, das ausgewählt ist aus den Metallen der 8. Gruppe des Periodensystems, die gegebenenfalls Metalle wie Cadmium, Cer, Bismut, Blei, Silber, Tellur oder Zinn als Beschleuniger enthalten.

## Claims

1. A process for preparing a hydroxyalkylated carbocyclic aromatic ether consisting of reacting said aromatic ether with a carbonyl compound in the presence of a catalyst, said process being **characterized in that** the hydroxyalkylation reaction is carried out in the presence of an effective quantity of a zeolite at a temperature of 100°C or less.

2. A process according to claim 1, **characterized in that** the carbocyclic aromatic ether has general formula (I):

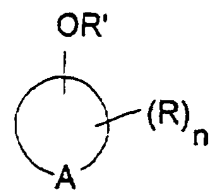

(I)

in which:

- A represents the residue of a cycle forming all or a portion of a monocyclic or polycyclic aromatic carbocyclic system containing at least one OR' group: said cyclic residue may carry one or more substituents;
- R represents one or more substituents, which may be identical or different;
- R' represents a hydrocarbon radical containing 1 to 24 carbon atoms, which may be a linear or branched, saturated or unsaturated acyclic aliphatic radical; a monocyclic or polycyclic, saturated, unsaturated or aromatic cycloaliphatic radical; or a linear or branched, saturated or unsaturated aliphatic radical carrying a cyclic substituent;
- R' and R may form a cycle which may contain a further heteroatom;
- n is a number less than or equal to 4.

3. A process according to claim 1 or claim 2, **characterized in that** the carbocyclic aromatic ether has general formula (I) in which R' represents:

- a linear or branched, saturated or unsaturated acyclic aliphatic radical, preferably a linear or branched alkyl radical containing 1 to 12 carbon atoms, preferably 1 to 6 carbon atoms: the hydrocarbon chain can optionally be interrupted by a heteroatom, a functional group and/or can optionally carry a substituent;
- a linear or branched, saturated or unsaturated acyclic aliphatic radical carrying a cyclic substituent which can optionally be substituted: said acyclic radical may be bonded to the cycle via a covalent bond, a heteroatom or a functional group;
- a carbocyclic radical which may be saturated or contain 1 or 2 unsaturated bonds in the cycle, generally containing 3 to 8 carbon atoms, preferably 6 carbon atoms in the cycle; said cycle may be substituted;
- an aromatic carbocyclic radical, preferably a monocyclic radical generally containing at least 4 carbon atoms, preferably 6 carbon atoms in the cycle; said cycle may be substituted.

4. A process according to claim 1, **characterized in that** the carbocyclic aromatic ether has general formula (I) in which R' represents a linear or branched alkyl radical containing 1 to 4 carbon atoms, preferably a methyl or phenyl radical.

5. A process according to any one of claims 1 to 4, **characterized in that** the carbocyclic aromatic ether has general formula (I) in which residue A represents the residue of a monocyclic aromatic carbocyclic compound containing at least 4 carbon atoms and preferably 6 carbon atoms or the residue of a polycyclic carbocyclic compound, preferably a naphthalene residue: residue A may carry one or more substituents on the aromatic ring.

6. A process according to any one of claims 1 to 5, **characterized in that** the carbocyclic aromatic ether has formula (Ia):

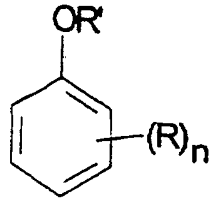

(Ia)

in which:

- n is a number less than or equal to 4, preferably 0, 1 or 2;
- radical R' represents a linear or branched alkyl radical containing 1 to 6 carbon atoms, preferably 1 to 4 carbon atoms, such as methyl, ethyl, propyl, isopropyl, butyl, isobutyl, sec-butyl, tert-butyl or a phenyl radical;
- radical(s) R represent one of the following atoms or groups:
  - a linear or branched alkyl radical containing 1 to 6 carbon atoms, preferably 1 to 4 carbon atoms, such as methyl, ethyl, propyl, isopropyl, butyl, isobutyl, sec-butyl or tert-butyl;
  - a linear or branched alkenyl radical containing 2 to 6 carbon atoms, preferably 2 to 4 carbon atoms, such as vinyl, allyl;
  - a cyclohexyl or benzyl radical;
  - a linear or branched alkoxy radical containing 1 to 6 carbon atoms, preferably 1 to 4 carbon atoms, such as a methoxy, ethoxy, propoxy, isopropoxy or butoxy radical;
  - an acyl group containing 2 to 6 carbon atoms;
  - a radical with formula:
    - $-R_1-OH$
    - $-R_1-COOR_2$
    - $-R_1-CHO$
    - $-R_1-NO_2$
    - $-R_1-CN$
    - $-R_1-N-(R_2)_2$
    - $-R_1-CO-N-(R_2)_2$
    - $-R_1-X$
    - $-R_1-CF_3$

    in which formulae $R_1$ represents a covalent bond or a linear or branched, saturated or unsaturated divalent hydrocarbon radical containing 1 to 6 carbon atoms, such as methylene, ethylene, propylene, isopropylene, or isopropylidene; radicals $R_2$, which may be identical or different, represent a hydrogen atom or a linear or branched alkyl radical containing 1 to 6 carbon atoms; and X represents a halogen atom, preferably a chlorine, bromine or fluorine atom;

- radicals R' and R and the 2 successive atoms on the benzene ring may form between them a cycle containing 5 to 7 carbon atoms, which can optionally contain a further heteroatom.

**7.** A process according to claim 6, **characterized in that** the carbocyclic aromatic ether has formula (Ia) where n is greater than or equal to 1, radicals R' and R and the 2 successive atoms of the benzene ring can be bonded together by an alkylene, alkenylene or alkenylidene radical containing 2 to 4 carbon atoms to form a saturated, unsaturated or aromatic heterocycle containing 5 to 7 carbon atoms in which one or more carbon atoms can be replaced by a heteroatom, preferably oxygen: radicals OR' and R preferably form a dioxymethylene or dioxyethylene radical.

**8.** A process according to claim 6 or claim 7, **characterized in that** the carbocyclic aromatic ether is an aromatic ether with formula (Ia) in which n equals 1, radical R' represents an alkyl radical containing 1 to 4 carbon atoms and R represents an alkoxy radical containing 1 to 4 carbon atoms or a hydroxy group.

**9.** A process according to claim 1 or claim 2. **characterized in that** the carbocyclic aromatic ether is anisole, phenetole, guaiacol, guetol, veratrol, 1,2-methylenedioxybenzene, or 2-methoxynaphthalene.

**10.** A process according to any one of claims 1 to 9, **characterized in that** the carbonyl compound has formula (II):

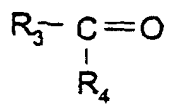

(II)

in which formula (II):

- $R_3$ and $R_4$, which may be identical or different, represent:
  - a hydrogen atom;
  - a linear or branched alkyl radical containing 1 to 6 carbon atoms;
  - a linear or branched alkenyl group containing 2 to 6 carbon atoms;
  - a phenyl group;
  - an electron withdrawing group.

**11.** A process according to claim 10, **characterized in that** the carbonyl compound has formula (II) in which the electron withdrawing group is selected from:

- a -CHO group;
- a $-COOR_5$ group in which $R_5$ represents a hydrogen atom or a linear or branched alkyl radical containing 1 to 6 carbon atoms;
- a $-CX_2H$ group in which X represents a halogen atom, preferably a chlorine atom;
- a $-CX_3$ group in which X represents a halogen atom, preferably a chlorine atom.

**12.** A process according to claim 10 or claim 11, **characterized in that** the carbonyl compound is selected from:

- formaldehyde or a formaldehyde generator such as trioxane or paraformaldehyde used in the form of linear polyformaldehydes of any degree of polymerisation, preferably containing 8 to 100 ($CH_2O$) units;
- glyoxylic acid;
- chloral;
- dichloroacetone;
- acetaldehyde;
- propionaldehyde;
- dichloroacetaldehyde;
- trichloroacetaldehyde;
- methyl pyruvate;
- ethyl pyruvate.

**13.** A process according to any one of claims 1 to 12, **characterized in that** the carbonyl compound is formaldehyde or a formaldehyde generator.

**14.** A process according to any one of claims 1 to 13, **characterized in that** the zeolite is a natural or synthetic zeolite.

**15.** A process according to claim 15, **characterized in that** the zeolite is a natural zeolite selected from: chabazite, clinoptilolite, erionite, mordenite, phillipsite, and offretite.

**16.** A process according to claim 15, **characterized in that** the zeolite is a synthetic zeolite selected from:

- synthetic zeolites with a one-dimensional network such as ZSM-4 zeolite, L zeolite, ZSM-12 zeolite, ZSM-22 zeolite, ZSM-23 zeolite, and ZSM-48 zeolite;
- zeolites with a two-dimensional network such as mordenite and ferrierite;

- zeolites with a three-dimensional network such as β zeolite, Y zeolite, X zeolite, ZSM-5 zeolite, ZSM-11 zeolite and offretite.

**17.** A process according to claim 16, **characterized in that** the zeolite is an H-β zeolite or an H-mordenite.

**18.** A process according to any one of claims 1 to 17, **characterized in that** the reaction is carried out in an aqueous medium or in the presence of an organic solvent selected from aliphatic and/or aromatic hydrocarbons which may be halogenated, preferably chlorinated, aliphatic, cycloaliphatic or aromatic ether-oxides, polar aprotic solvents, preferably nitrogen-containing compounds, aliphatic or aromatic nitrites, tetramethylenesulphone, or dimethylsulphoxide.

**19.** A process according to any one of claims 1 to 18, **characterized in that** the ratio between the number of moles of carbonyl compound and the number of moles of aromatic ether is between 1 and 50; preferably 1 to 25 when natural or synthetic zeolites are used such as L, X, Y zeolites, mordenites with a Si/Al molar ratio of 5 to 12, ferrierite and offretite; preferably 8 to 25 when synthetic zeolites are used such as ZSM-4 zeolite, ZSM-5 zeolite, ZSM-11 zeolite, ZSM-12 zeolite, ZSM-22 zeolite, ZSM-23 zeolite, ZSM-48 zeolite, L zeolite, β zeolite and mordenites with a Si/Al molar ratio of 12 to 150.

**20.** A process according to any one of claims 1 to 19, **characterized in that** the quantity of catalyst is 5% to 80%, preferably 10% to 50% by weight with respect to the aromatic ether used.

**21.** A process according to any one of claims 1 to 20, **characterized in that** the temperature at which the hydroxyalkylation reaction is carried out is 100°C or less, preferably between 20°C and 100°C, more preferably between 40°C and 90°C.

**22.** A process according to any one of claims 1 to 21, **characterized in that** the catalyst, carbonyl compound with formula (II), and optional organic solvent is charged then the carbocyclic aromatic ether is introduced continuously or in fractions and the reaction mixture is then heated to the desired temperature, or the reaction is carried out continuously in a tube reactor comprising the solid catalyst disposed in a fixed bed.

**23.** A process according to claim 22, **characterized in that** at the end of the reaction, a liquid phase comprising the hydroxyalkylated aromatic ether is recovered and extracted from an organic solvent then the organic phase obtained is either directly oxidised, or counter-extracted with a basic aqueous solution to obtain an aqueous phase containing the product in salt form which is then oxidised.

**24.** A process according to claim 23, **characterized in that** the extraction solvent is selected from halogenated aliphatic or aromatic hydrocarbons such as such as chloromethane, dichloromethane, 1,2-dichloroethane, chlorobenzene, dichlorobenzenes, or trichlorobenzenes; esters such as butyl, isopropyl, amyl, or cyclohexyl acetates; and ketones such as acetone, methylethylketone, or methylisobutylketone.

**25.** A process according to any one of claims 22 to 24, **characterized in that** the aqueous counter-extraction solution is an aqueous sodium or potassium hydroxide solution: the proportion of mineral base used is in the range 0.5 moles to 3 moles of sodium or potassium hydroxide with respect to the hydroxyalkylated aromatic ether to be oxidised.

**26.** A process for oxidising a hydroxyalkylated aromatic ether, **characterized in that** it consists of:

- preparing said compound using the process defined in any one of claims 1 to 25, by reacting an aromatic ether with a carbonyl compound at a temperature of 100°C or less in the presence of an effective quantity of a zeolite;
- recovering said hydroxyalkylated aromatic ether in the liquid phase then oxidising it using molecular oxygen or a gas containing molecular oxygen in the presence of a catalyst based on a metal M selected from the metals in group 8 of the periodic table, optionally comprising metals such as cadmium, cerium, bismuth, lead, silver, tellurium or tin as activators.

**27.** A process according to claim 26, **characterized in that** the catalyst is based on nickel, ruthenium, rhodium, palladium, osmium, indium, platinum or mixtures thereof, preferably based on platinum and/or palladium.

**28.** A process according to claim 26 or claim 27, **characterized in that** the platinum catalyst is supplied in the form

of platinum black, platinum oxide, or the noble metal itself deposited on various supports such as carbon black, calcium carbonate, aluminas and activated silicas or equivalent materials, preferably carbon black.

**29.** A process according to any one of claims 26 to 28, **characterized in that** the quantity of catalyst to be used, expressed as the weight of metal M with respect to that of the hydroxyalkylated aromatic ether, is between 0.01% and 4%, preferably between 0.04% and 2%.

**30.** A process according to any one of claims 26 to 29, **characterized in that** the activator is an organic or mineral derivative of bismuth selected from the group formed by: bismuth oxides; bismuth hydroxides; bismuth or bismuthyl salts of mineral hydracids, preferably chloride, bromide, iodide, sulphide, selenide, or telluride; bismuth or bismuthyl salts of mineral oxyacids, preferably the sulphite, sulphate, nitrite, nitrate, phosphite, phosphate, pyrophosphate, carbonate, perchlorate, antimonate, arsenate, selenite or selenate; bismuth or bismuthyl salts of aliphatic or aromatic organic acids, preferably the acetate, propionate, salicylate, benzoate, oxalate, tartrate, lactate, citrate; and bismuth or bismuthyl phenates, preferably the gallate or pyrogallate.

**31.** A process according to claim 30, **characterized in that** the bismuth derivative is selected from the group formed by: bismuth oxides $Bi_2O_3$ and $Bi_2O_4$; bismuth hydroxide $Bi(OH)_3$; bismuth chloride $BiCl_3$; bismuth bromide $BiBr_3$; bismuth iodide $BiI_3$; neutral bismuth sulphate $Bi_2(SO_4)_3$; neutral bismuth nitrate $Bi(NO_3)_3,5H_2O$; bismuthyl nitrate $BiO(NO_3)$; bismuthyl carbonate $(BiO)_2CO_3,0.5H_2O$; bismuth acetate $Bi(C_2H_3O_2)_3$ and bismuth salicylate $C_6H_4CO_2(BiO)OH$.

**32.** A process according to any one of claims 26 to 31, **characterized in that** the quantity of activator is selected so that the medium is supplied with: at least 0.1% by weight of metal activator with respect to the weight of metal M used, and 10 to 900 ppm by weight of metal M with respect to the hydroxyalkylated aromatic ether.

**33.** A process according to any one of claims 26 to 32, **characterized in that** the oxidation reaction is carried out within a temperature range of 45°C to 100°C, more preferably between 60°C and 80°C.

**34.** A process for preparing vanillin by oxidising 3-methoxy-4-hydroxybenzyl alcohol, **characterized in that** the 3-methoxy-4-hydroxybenzyl alcohol is prepared by hydroxymethylation of guaiacol carried out in the presence of an effective quantity of a zeolite at a temperature of 100°C or less using the process described in any one of claims 1 to 25.

**35.** A process according to claim 34, **characterized in that** the 3-methoxy-4-hydroxybenzyl alcohol is recovered in the liquid phase then is oxidised using the process defined in any one of claims 26 to 33 using molecular oxygen or a gas containing molecular oxygen in the presence of a catalyst based on a metal M selected from metals from group 8 of the periodic table optionally containing metals such as cadmium, cerium, bismuth, lead, silver, tellurium or tin as activators.

**36.** A process for preparing ethylvanillin by oxidising 3-ethoxy-4-hydroxybenzyl alcohol, **characterized in that** the 3-ethoxy-4-hydroxybenzyl alcohol is obtained by hydroxymethylation of guetol carried out in the presence of an effective quantity of a zeolite at a temperature of 100°C or less using the process described in any one of claims 1 to 25.

**37.** A process according to claim 36, **characterized in that** the 3-ethoxy-4-hydroxybenzyl alcohol is recovered in the liquid phase then is oxidised using the process defined in any one of claims 26 to 33 using molecular oxygen or a gas containing molecular oxygen in the presence of a catalyst based on a metal M selected from metals from group 8 of the periodic table optionally containing metals such as cadmium, cerium, bismuth, lead, silver, tellurium or tin as activators.